# EUROPEAN PATENT SPECIFICATION

(11) **EP 4 097 145 B1**
(45) Date of publication and mention of the grant of the patent: **28.01.2026**
(21) Application number: 21703182.2
(22) Date of filing: 29.01.2021
(51) Int. Cl.: C07K 17/14, C07K 17/02

(54) **REDUCTION OF ADSORPTION**
VERRINGERUNG DER ADSORPTION
RÉDUCTION D'ADSORPTION

(30) Priority: 30.01.2020 EP 20154584
(43) Date of publication of application: 07.12.2022
(73) Proprietor: LEUKOCARE AG, 82152 Martinsried / München (DE)
(72) Inventor: GEISSLER, Andreas, 81375 München (DE); KEMTER, Kristina, 85748 Garching (DE); HAUCK, Sabine, 80801 München (DE); BÖCKEL, Maximilian Maarten, 82152 Martinsried (DE)
(74) Representative: Vossius & Partner Patentanwälte Rechtsanwälte mbB
(86) International application number: PCT/EP2021/052153
(87) International publication number: WO 2021/152121

(56) References cited:
- EP-A1- 2 851 083
- WO-A1-2007/128550
- WO-A1-2013/001044
- WO-A1-2018/154319
- WO-A2-2007/092772
- WO-A2-2009/086400
- YUI SHIKIYA ET AL: "Arginine Inhibits Adsorption of Proteins on Polystyrene Surface", PLOS ONE, 1 August 2013 (2013-08-01), pages 1 - 7, XP055567864, Retrieved from the Internet <URL:https://www.ncbi.nlm.nih.gov/pmc/articles/PMC3742611/pdf/pone.0070762.pdf> [retrieved on 20190312], DOI: 10.1371/journal.pone.0070762
- PRISCILLA KHEDDO ET AL: "Characterizing monoclonal antibody formulations in arginine glutamate solutions using 1 H NMR spectroscopy", MABS, vol. 8, no. 7, 2 September 2016 (2016-09-02), US, pages 1245 - 1258, XP055643123, ISSN: 1942-0862, DOI: 10.1080/19420862.2016.1214786

## Description

The present invention is defined by the claims. Accordingly, the invention relates to a method of reducing or preventing the adsorption of a polypeptide on a surface, or the aggregation of a polypeptide in a liquid composition in contact with a surface comprising the steps of a) providing a composition comprising the polypeptide and at least two different amino acids in a total amino acid concentration from 10 mM to 550 mM; where the composition comprises at least two different amino acids wherein at least one amino acid is selected from group (d) of amino acids with negatively charged R groups; and further comprises at least one amino acid selected is from group (e) of amino acids with aromatic R groups; and b) contacting the composition with the surface, wherein the surface comprises or consists of a metal, a glass or a polymer material, preferably selected from polyethylene (PE), for example selected from polyethylene terephthalate (PET), high density polyethylene (HDPE), low-density polyethylene (LDPE), linear low density polyethylene (LLDPE), polypropylene (PP), polystyrene (PS), polyvinylchloride (PVC), polyvinylidene chloride (PVDC), polytetrafluorethylene (PTFE), polyethersulphone (PES), polymethylmethacrylate, polycarbonate (PC, bisphenol A), nylon, polyether urethane, polysiloxane (silicone), polychlorotrifluoroethylene (PCTFE)/PVC laminates, polyamide (PA), e.g. orientated polyamide (OPA), cyclic olefin copolymer, or mixtures and copolymers thereof.

### Background

Recombinant biomolecules are commonly used for the treatment of diseases in a wide range of indications. Recombinant biomolecules include therapeutic antibodies, coagulation factors or proteins comprised in therapeutic viruses. From recombinant expression of the respective biomolecule in a host cell to their site of action in the body of a patient, the biomolecules encounter interaction with many different surfaces. In addition to surfaces of equipment used for isolating in purifying the biomolecules, the biomolecules are exposed to surfaces of containers used for storing and surfaces of medical devices, or parts thereof, used for administering the biomolecules, such as for examples syringes, pumps, tubing, containers, bags, and needles.

Due to their surface activity, protein molecules are able to interact with various surfaces. These interactions often result in an adsorption of the molecule on the respective surface. Thus, the molecules are withdrawn from the composition administered to a patient, resulting in a loss of therapeutic potency of a composition comprising therapeutic biomolecules. Especially in compositions with a low concentration of therapeutic biomolecules, the ratio of protein adsorbed to a surface to solubilized protein may be particularly high. Therefore, a higher concentration of biomolecules may be required in the composition to reach the desired potency in the patient. Loss of protein at the liquid/solid surface interface is also an important consideration and may be particularly relevant during dilution and administration that involve the use of plastic bags and intravenous lines. Adsorption of mAbs to glass vials has been shown to be due predominantly to electrostatic interactions (Couston RG, Skoda MW, Uddin S, van der Walle CF. Adsorption behaviour of a human monoclonal antibody at hydrophilic and hydrophobic surfaces. MAbs. 2013;5(1):126-139).

Further to the loss of potency by the amount adsorbed on the surface, surface adsorption-desorption of misfolded protein that reveal hydrophobic residues, and thereby facilitate protein aggregation through hydrophobic interactions, may result in formation of soluble aggregates (oligomers), which in turn may nucleate further protein aggregation, ultimately generating visible particles in solution (Couston RG, Skoda MW, Uddin S, van der Walle CF. Adsorption behaviour of a human monoclonal antibody at hydrophilic and hydrophobic surfaces. MAbs. 2013;5(1):126-139). Especially highly concentrated biomolecules are susceptible to aggregation induced by surface interaction. Further to the loss of potency, aggregates of biomolecules promote the generation of anti-dug-antibodies in subjects to which therapeutic biomolecule formulations comprising aggregates are administered.

The properties of the surface, such as functional groups on the surface, charge and hydrophobicity all influence the interaction of the biomolecule with the surface. The most important driving forces for surface adsorption of proteins are hydrophobic interactions, electrostatic interactions and protein restructuring, but changes in hydrogen bonding and van de Waals forces are to be considered likewise. Most proteins adsorb to hydrophobic surfaces to some extent. Adsorption generally increases with the hydrophobicity of either the protein or the surface (Pinholt C, Hartvig RA, Medlicott NJ, Jorgensen L. The importance of interfaces in protein drug delivery - why is protein adsorption of interest in pharmaceutical formulations?. Expert Opin Drug Deliv. 2011;8(7):949-964). Furthermore, proteins tend to absorb rather on charged than on uncharged surfaces. Additionally, the topography of the surface, especially its roughness, may further strongly influence protein adsorption (Migliorini E, Weidenhaupt M, Picart C. Practical guide to characterize biomolecule adsorption on solid surfaces. Biointerphases. 2018;13(6):06D303).

To avoid adsorption of the protein of interest, usually a therapeutic protein, additional proteins, especially albumins such as e.g. HSA, BSA etc., may be added to a therapeutic protein or peptide solutions in order to avoid the adsorption to surfaces, unnecessary agitation and to minimize unnecessary air- or foam formation. However, the addition of BSA and HSA is associated with the risk of viral contamination.

To prevent adsorption and related aggregation, also surfactants such as polysorbates are commonly used in protein bioprocessing, formulation and delivery. In general, polysorbates compete with the protein for an interface and adsorb to exposed hydrophobic patches on the protein surface. Non-ionic surfactants such as polysorbate 20 and 80 (Tween^{®} 20 and 80) and the polyethylene glycol-polypropylene glycol-polyethylene glycol (PEO-PPO-PEO) triblock co-polymers (Pluronics^{®}) are frequently used compounds from this class of excipients (Couston RG, Skoda MW, Uddin S, van der Walle CF. Adsorption behavior of a human monoclonal antibody at hydrophilic and hydrophobic surfaces. MAbs. 2013;5(1):126-139). Compositions comprising a polysorbate, a sugar and a combination of the polar amino acids glutamine, asparagine with the negatively charged amino acids glutamate and aspartate is disclosed in WO 2005/007185 A2. However, polysorbates are susceptible to hydrolysis or oxidation, and the resulting degradation products promote aggregation and/or reduce solubility of the protein and destabilize protein formulations. Due to their micelle forming properties polysorbates may cause problems during the manufacturing process of recombinant biomolecules when filters are used in the downstream processing.

The use of surfactants or proteins to reduce surface adsorption is also disclosed in WO 2013/001044 A1.

Shikiya and colleagues demonstrated that nonspecific adsorption of protein to polystyrene particles (PS particles) could be reduced by supplementing the protein solutions with an excipient selected from arginine, lysine, or guanidinium (Shikiya Y, Tomita S, Arakawa T, Shiraki K. Arginine inhibits adsorption of proteins on polystyrene surface. *PLoS One,* 8 (8), e70762). However, to maintain a significant activity of the protein, high concentrations of 500 mM of the single excipients were required.

WO 2018/050870 A1 discloses the use of compositions comprising least three amino acids and a sugar in the different process steps of a biopharmaceutical drug production including harvesting, purification, re-buffering, enrichment, freezing, thawing, and filling. However, the document does not address the problem of adsorption of proteins to surfaces, or to the interaction of biomolecules and medical devices used for administering biomolecules.

WO 2007/128550 A1 discloses a method wherein biomolecules are coupled to a surface to produce a solid coated carrier carrying biological material. In the disclosed method biomolecules are attached to the carrier by incubating the carrier with an aqueous solution containing the biomolecule and subsequently removing the aqueous solution. After the biomolecules have already been coupled to the carrier, the attached biomolecules are incubated with a solution comprising amino acids. Accordingly, the disclosed method does not relate to a method of reducing or preventing the adsorption of a polypeptide but to the opposite thereof, at the stabilisation of a biomolecule bound to a surface.

There is a need to overcome the above described problems and shortcomings of the prior art.

### Description

The problem is solved by the invention according to the amended claims and as further described herein.

In a first aspect the present invention relates to a method of reducing or preventing the adsorption of a polypeptide on a surface comprising the steps of:
a) providing a composition comprising the polypeptide and at least two different amino acids in a total amino acid concentration from 10 mM to 550 mM; where the composition comprises at least two different amino acids wherein at least one amino acid is selected from group (d) of amino acids with negatively charged R groups; and further comprises at least one amino acid selected is from group (e) of amino acids with aromatic R groups; and
b) contacting the composition with the surface, wherein the surface comprises or consists of a metal, a glass or a polymer material, preferably selected from polyethylene (PE), for example selected from polyethylene terephthalate (PET), high density polyethylene (HDPE), low-density polyethylene (LDPE), linear low density polyethylene (LLDPE), polypropylene (PP), polystyrene (PS), polyvinylchloride (PVC), polyvinylidene chloride (PVDC), polytetrafluorethylene (PTFE), polyethersulphone (PES), polymethylmethacrylate, polycarbonate (PC, bisphenol A), nylon, polyether urethane, polysiloxane (silicone), polychlorotrifluoroethylene (PCTFE)/PVC laminates, polyamide (PA), e.g. orientated polyamide (OPA), cyclic olefin copolymer, or mixtures and copolymers thereof..

As shown in the examples 1.2 to 1.6, the inventors surprisingly found that compositions comprising only two different amino acids effectively reduce adsorption of a polypeptide on a surface.

The term "polypeptide" in accordance with the present invention describes a group of molecules which comprises the group of peptides, consisting of up to 30 amino acids, as well as the group of polypeptides, consisting of more than 30 amino acids. Also encompassed by the term "polypeptide" are proteins as well as fragments of proteins. Polypeptides may form dimers, trimers and higher oligomers, i.e. consisting of more than one polypeptide molecule. Polypeptide molecules forming such dimers, trimers etc. may be identical or non-identical. The corresponding higher order structures are, consequently, termed homo- or heterodimers, homo- or heterotrimers etc. Homo- or heterodimers etc. also fall under the definition of the term "polypeptide". The terms "polypeptide" and "protein" are used interchangeably herein. The term "polypeptide" also refers to naturally modified polypeptides wherein the modification is effected e.g. by post-translational modifications such as e.g. glycosylation, acetylation, phosphorylation and the like. Such modifications are well known in the art.

Preferably, the polypeptide according to the present invention is a polypeptide selected from the group consisting of therapeutic proteins, like antibodies, growth factors, cytokines, protein or peptide hormones, growth hormones, blood factors, therapeutic enzymes, therapeutic vaccines or fragments thereof which retain their biological activity.

An antibody in accordance with the present invention may be for example a polyclonal or monoclonal antibody. The term "antibody", as used herein, also includes embodiments such as chimeric (human constant domain, non-human variable domain), single chain and humanized (human antibody with the exception of non-human CDRs) antibodies, as well as antibody fragments, like, inter alia, Fab, Fab', Fd, F(ab')2, Fv or scFv fragments or nanobodies, i.e. single monomeric variable antibody domains; see, for example, Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

In accordance with an even more preferred embodiment of the invention, the antibody is a therapeutic antibody.

The term "therapeutic antibody" as used herein, describes monoclonal antibodies (mAb) binding monospecifically to certain cells or proteins, e.g. in order to stimulate the immune system and/or to attack cells of a patient for therapeutic purposes.

Non-limiting examples of preferred antibodies and in particular therapeutic antibodies include Infliximab, Bevacizumab, Ranibizumab, Cetuximab, Ranibizumab, Palivizumab, Abagovomab, Abciximab, Actoxumab, Adalimumab, Afelimomab, Afutuzumab, Alacizumab, Alacizumab pegol, ALD518, Alemtuzumab, Alirocumab, Alemtuzumab, Altumomab, Amatuximab, Anatumomab mafenatox, Anrukinzumab, Apolizumab, Arcitumomab, Aselizumab, Altinumab, Atlizumab, Atorolimiumab, tocilizumab, Bapineuzumab, Basiliximab, Bavituximab, Bectumomab, Belimumab, Benralizumab, Bertilimumab, Besilesomab, Bevacizumab, Bezlotoxumab, Biciromab, Bivatuzumab, Bivatuzumab mertansine, Blinatumomab, Blosozumab, Brentuximab vedotin, Briakinumab, Brodalumab, Canakinumab, Cantuzumab mertansine, Cantuzumab mertansine, Caplacizumab, Capromab pendetide, Carlumab, Catumaxomab, CC49, Cedelizumab, Certolizumab pegol, Cetuximab, Citatuzumab bogatox, Cixutumumab, Clazakizumab, Clenoliximab, Clivatuzumab tetraxetan, Conatumumab, Crenezumab, CR6261 , Dacetuzumab, Daclizumab, Dalotuzumab, Daratumumab, Demcizumab, Denosumab, Detumomab, Dorlimomab aritox, Drozitumab, Duligotumab, Dupilumab, Ecromeximab, Eculizumab, Edobacomab, Edrecolomab, Efalizumab, Efungumab, Elotuzumab, Elsilimomab, Enavatuzumab, Enlimomab pegol, Enokizumab, Enokizumab, Enoticumab, Enoticumab, Ensituximab, Epitumomab cituxetan, Epratuzumab, Erlizumab, Ertumaxomab, Etaracizumab, Etrolizumab, Exbivirumab, Exbivirumab, Fanolesomab, Faralimomab, Farletuzumab, Fasinumab, FBTA05, Felvizumab, Fezakinumab, Ficlatuzumab, Figitumumab, Flanvotumab, Fontolizumab, Foralumab, Foravirumab, Fresolimumab, Fulranumab, Futuximab, Galiximab, Ganitumab, Gantenerumab, Gavilimomab, Gemtuzumab ozogamicin, Gevokizumab, Girentuximab, Glembatumumab vedotin, Golimumab, Gomiliximab, GS6624, Ibalizumab, Ibritumomab tiuxetan, Icrucumab, Igovomab, Imciromab, Imgatuzumab, Inclacumab, Indatuximab ravtansine, Infliximab, Intetumumab, Inolimomab, Inotuzumab ozogamicin, Ipilimumab, Iratumumab, Itolizumab, Ixekizumab, Keliximab, Labetuzumab, Lebrikizumab, Lemalesomab, Lerdelimumab, Lexatumumab, Libivirumab, Ligelizumab, Lintuzumab, Lirilumab, Lorvotuzumab mertansine, Lucatumumab, Lumiliximab, Mapatumumab, Maslimomab, Mavrilimumab, Matuzumab, Mepolizumab, etelimumab, Milatuzumab, Minretumomab, Mitumomab, Mogamulizumab, Morolimumab, Motavizumab, Moxetumomab pasudotox, Muromonab-CD3, Nacolomab tafenatox, Namilumab, Naptumomab estafenatox, Namatumab, Natalizumab, Nebacumab, Necitumumab, Nerelimomab, Nesvacumab, Nimotuzumab, Nivolumab, Nofetumomab merpentan, Ocaratuzumab, Ocrelizumab, Odulimomab, Ofatumumab, Olaratumab, Olokizumab, Omalizumab, Onartuzumab, Oportuzumab monatox, Oregovomab, Orticumab, Otelixizumab, Oxelumab, Ozanezumab, Ozoralizumab, Pagibaximab, Palivizumab, Panitumumab, Panobacumab, Parsatuzumab, Pascolizumab, Pateclizumab, Patritumab, Pemtumomab, Perakizumab, Pertuzumab, Pexelizumab, Pidilizumab, Pintumomab, Placulumab, Ponezumab, Priliximab, Pritumumab, PRO140, Quilizumab, Racotumomab, Radretumab, Rafivirumab, Ramucirumab, Ranibizumab, Raxibacumab, Regavirumab, Reslizumab, Rilotumumab, Rituximab, Robatumumab, Roledumab, Romosozumab, Rontalizumab, Rovelizumab, Ruplizumab, Samalizumab, Sarilumab, Satumomab pendetide, Secukinumab, Sevirumab, Sibrotuzumab, Sifalimumab, Siltuximab, Simtuzumab, Siplizumab, Sirukumab, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tanezumab, Taplitumomab paptox, Tefibazumab, Telimomab aritox, Tenatumomab, Tefibazumab, Telimomab aritox, Tenatumomab, Teneliximab, Teplizumab, Teprotumumab, TGN1412, Tremelimumab, Ticilimumab, Tildrakizumab, Tigatuzumab, TNX-650, Tocilizumab, Toralizumab, Tositumomab, Tralokinumab, Trastuzumab, TRBS07, Tregalizumab, Tremelimumab, Tucotuzumab celmoleukin, Tuvirumab, Ubiituximab, Urelumab, Urtoxazumab, Ustekinumab, Vapaliximab, Vatelizumab, Vedolizumab, Veltuzumab, Vepalimomab, Vesencumab, Visilizumab, Volociximab, Vorsetuzumab mafodotin, Votumumab, Zalutumumab, Zanolimumab, Zatuximab, Ziralimumab and Zolimomab aritox.

In accordance with another embodiment of the invention, the antibody is a diagnostic antibody.

The term "diagnostic antibody", as it is used herein, describes antibodies that are conjugated e.g. with a radiolabel, fluorescent label, or colour-forming enzyme and are used as a "probe" to biologic target structures for diagnostic purposes. Possible applications include pregnancy tests, immunoblotting, ELISA and immunohistochemical staining without being limiting.

The term "hormones", as used herein, is well known in the art and relates to a group of therapeutic biomolecules used for the treatment of metabolism disorders. Non-limiting examples include teriparatide or estrogen. Teriparatide is a recombinant form of the growth hormone parathyroid hormone that is commonly used for the treatment of impaired bone metabolism such as osteoporosis. Estrogen is commonly used for the therapy of menopausal disorders and is given in conjunction with progesterone to reduce the risk for uterine cancer.

According to the present invention the polypeptide may be comprised in higher oligomers, alone or with other polypeptide species. Preferably, the polypeptide and oligomers thereof may be comprised in the capsid of a virus or a viral vector.

The term "viral vector", in accordance with the present invention, relates to a carrier, i.e. a "vector" that is derived from a virus. "Viral vectors" in accordance with the present invention include vectors derived from naturally occurring or modified viruses, as well as virus like particles (VLPs). "Viral vector" may be viruses derived from naturally occurring viruses by genetic modification.

Accordingly, viruses or viral vectors already available in the art, as well as novel viral vectors, may be employed in the claimed method. Thus, in a specific aspect the invention relates to a method of reducing or preventing the adsorption of a virus or viral vector on a surface comprising the steps of:
a) providing a composition comprising the virus or viral vector and at least two different amino acids in a total amino acid concentration from 10 mM to 550 mM; where the composition comprises at least two different amino acids wherein at least one amino acid is selected from group (d) of amino acids with negatively charged R groups; and further comprises at least one amino acid selected is from group (e) of amino acids with aromatic R groups; and
b) contacting the composition with the surface, wherein the surface comprises or consists of a metal, a glass or a polymer material, preferably selected from polyethylene (PE), for example selected from polyethylene terephthalate (PET), high density polyethylene (HDPE), low-density polyethylene (LDPE), linear low density polyethylene (LLDPE), polypropylene (PP), polystyrene (PS), polyvinylchloride (PVC), polyvinylidene chloride (PVDC), polytetrafluorethylene (PTFE), polyethersulphone (PES), polymethylmethacrylate, polycarbonate (PC, bisphenol A), nylon, polyether urethane, polysiloxane (silicone), polychlorotrifluoroethylene (PCTFE)/PVC laminates, polyamide (PA), e.g. orientated polyamide (OPA), cyclic olefin copolymer, or mixtures and copolymers thereof.

Preferably, the viral vectors are selected from the group consisting of modified vaccinia Ankara virus (MVA), adenovirus, adeno associated virus, lentivirus, Vesicular stomatitis virus (VSV), herpes simplex virus, or measles virus. Most preferably, the viral vector is adeno associated virus, lentivirus or adenovirus.

The virus or viral vector may be comprised in a therapeutic vaccine. The term "therapeutic vaccines", in accordance with the present invention, relates to the immunogenic parts of an attenuated or killed pathogen (antigen), the antigenic components of virus lysates or a recombinantly produced individual proteinic virus antigens.

Preferably, the composition according to the invention is an aqueous solution. The term "aqueous solution", as used herein, is well known to the person skilled in the art and relates to a solution in which the solvent is predominantly water. An "aqueous solution" may also comprise non-aqueous solvents, such as organic solvents, to a small amount.

The term "amino acid", in accordance with the present invention, relates to organic molecules that have a carboxylic acid group, an amino group and a side-chain that varies between different amino acids. Amino acids are the essential building blocks of proteins. In accordance with the present invention, the term "amino acid" refers to free amino acids which are not bound to each other to form oligo- or polymers such as dipeptides, tripeptides, oligopeptides or polypeptide.

The amino acids comprised in the composition according to the present invention can be selected from naturally occurring amino acids as well as artificial amino acids or derivatives of these naturally occurring or artificial amino acids.

Naturally occurring amino acids are e. g. the 20 proteinogenic amino acids glycine, proline, arginine, alanine, asparagine, aspartic acid, glutamic acid, glutamine, cysteine, phenylanine, lysine, leucine, isoleucine, histidine, methionine, serine, valine, tyrosine, threonine and tryptophan. Other naturally occurring amino acids are e. g. carnitine, creatine, creatinine, guanidinoacetic acid, ornithine, hydroxyproline, homocysteine, citruliine, hydroxylysine or beta-alanine.

Artificial amino acids are amino acids that have a different side chain length and/or side chain structure and/or have the amino group at a site different from the alpha-C-atom. Derivates of amino acids are modified amino acids, including, without being limiting, n-acetyl-tryptophan, phosphonoserine, phosphonothreonine, phosphonotyrosine, melanin, argininosuccinic acid and salts thereof and DOPA.

In connection with the present invention, all the terms also include the salts of the respective amino acids.

In accordance with the present invention, the composition comprises at least two different amino acids in a total amino acid concentration from 10 mM to 550 mM; where the composition comprises at least two different amino acids wherein at least one amino acid is selected from group (d) of amino acids with negatively charged R groups; and further comprises at least one amino acid selected is from group (e) of amino acids with aromatic R groups. For example, the term "at least two different amino acids" also relates to at least three different amino acids, such as at least four, at least five, at least six, at least seven, at least eight, at least nine, at least ten different amino acids or more, such as at least eleven, at least 12, at least 13, at least 14, at least 15, at least 16, at least 17 or at least 18 different amino acids. The term further encompasses exactly two, three, exactly four, exactly five, exactly six, exactly seven, exactly eight, exactly nine, exactly ten, exactly eleven, exactly 12, exactly 13, exactly 14, exactly 15, exactly 16, exactly 17 or exactly 18 different amino.

The term "different amino acids" as used herein relates to the presence of at least two different amino acid species. Of each species a number of individual molecules of the respective amino acid species will be present depending on the given concentration of the respective amino acid species.

The term "total amino acid concentration" of a composition describes the sum of all concentrations of single amino acid species comprised in a composition. For example, a composition comprising 100 mM glycine, 100 mM arginine, and 300 mM valine has a total amino acid concentration of 300 mM.

The composition may also comprise at least one dipeptide and/or tripeptide. The term "dipeptide or tripeptide", as used herein, relates to peptides consisting of two or three amino acids, respectively. Exemplary dipeptides are glycylglutamine (Gly-Gln, giving rise to an enhanced stability as compared to glutamine alone), glycyltyrosine (Gly-Tyr, giving rise to an increased solubility in water as compared to tyrosine alone), alanylglutamine (Ala-Gln, giving rise to an increased solubility in water as compared to glutamine alone) and glycylglycine.

Further non-limiting examples of naturally occurring dipeptides are carnosine (beta-alanyl-L-histidine), N-acetyl-carnosine (N-acetyl-(beta-alanyl-L-histidine), anserine (beta-alanyl-N-methyl histidine), homoanserine (N-(4-aminobutyryl)-L-histidine), kyotorphin (L-tyrosyl-L-arginine), balenine (or ophidine) (beta-alanyl-N tau-methyl histidine), glorin (N- propionyl-y-L-glutamyl-L-ornithine-6-lac ethyl ester) and barettin (cyclo-[(6-bromo-8-en-tryptophan)-arginine}).Examples of artificial dipeptides include, without being limiting, aspartame (N-L-a-aspartyl-L-phenylalanine 1-methyl ester) and pseudoproline.

Exemplary tripeptides are giutathione (y-glutamyl-cysteinyl-glycine) and its analogues ophthalmic acid (L-y-glutamyl-L-a-aminobutyryl-glycine) as well as norophthalmic acid (y-glutamyl-alanyl-glycine). Further non-limiting examples of tripeptides include isoleucine-proline-proline (IPP), glypromate (Gly-Pro-Glu), thyrotropin-releasing hormone (TRH, thyroliberin or protirelin) (L-pyroglutamyl-L-histidinyl-L-prolinamide), melanostatin (prolyl-leucyl-glycinamide), leupeptin (N-acetyl-L-leucyl-L-leucyl-L-argininal) and eisenin (pGlu-GIn-Ala-OH). It is preferred that the at least one di- or tripeptide and more preferred all di- or tripeptides, when used in connection with medical applications, do not exert any pharmacological properties.

Preferably, at least one dipeptide is selected from the group consisting of carnosin, glycyltryrosine, glycylglycine and glycylglutamine.

In accordance with the present invention, the at least two amino acids are selected from the at least the two different groups (d) and (e). In other words, in a further preferred embodiment, when three amino acids are comprised in the composition, the three amino acids may be selected from the two different groups (d) and (e) and at least one different group of (a) to (c). The same consideration applies when four amino acids are comprised in the composition, in which case the amino acids have to be from groups (d), (e) and at least one different group selected from (a) to (c), more preferably from at least two different groups selected from (a) to (c) and most preferably from all of the different groups (a) to (c). Inter alia, when five amino acids are comprised in the composition, the amino acids have to be from (d), (e) and at least one different group selected from (a) to (c), more preferably from at least two different groups (a) to (c), and most preferably from three different groups (a) to (c). The same considerations apply when more than five amino acids are comprised in the composition, such as e.g. six or seven amino acids, in which case these amino acids are selected from (d), (e) and at least three different groups selected from (a) to (c), more preferably from at least two different groups (a) to (c), and most preferably from all three different groups (a) to (c).

In another preferred embodiment of the invention, the composition comprises at least one amino acid selected from each group of (a) an amino acid with non-polar, aliphatic R groups; (b) an amino acid with polar, uncharged R groups; (c) an amino acid with positively charged R groups; (d) an amino acid with negatively charged R groups and (e) an amino acid with aromatic R groups.

In one embodiment, the composition comprises only amino acids selected from no more than the two different groups (d) and (e). In a further embodiment the composition comprises only amino acids selected from no more than three different groups (d), (e) and one of (a) to (c).

According to the invention group (a) of amino acids with non-polar, aliphatic R groups may comprise glycine, alanine, isoleucine, leucine, valine, and proline; group (b) of amino acids with polar, uncharged R group may comprise glutamine, asparagine, serine, threonine, methionine, cysteine; group (c) of amino acids with positively charged R groups may comprise arginine, lysine, and histidine; group (d) of amino acids with negatively charged R groups may comprise aspartic acid and glutamic acid; and group (e) of amino acids with aromatic R groups may comprise phenylalanine, tryptophane, tyrosine, and histidine.

In accordance with the invention, the composition comprises at least two different amino acids wherein at least one amino acid is selected from group (e) of amino acids with negatively charged R groups; and at least one amino acid is selected from group (e) of amino acids acid with aromatic R groups. The composition may also comprise no other amino acids than one amino acid selected group (d) and one amino acid selected from group (e).

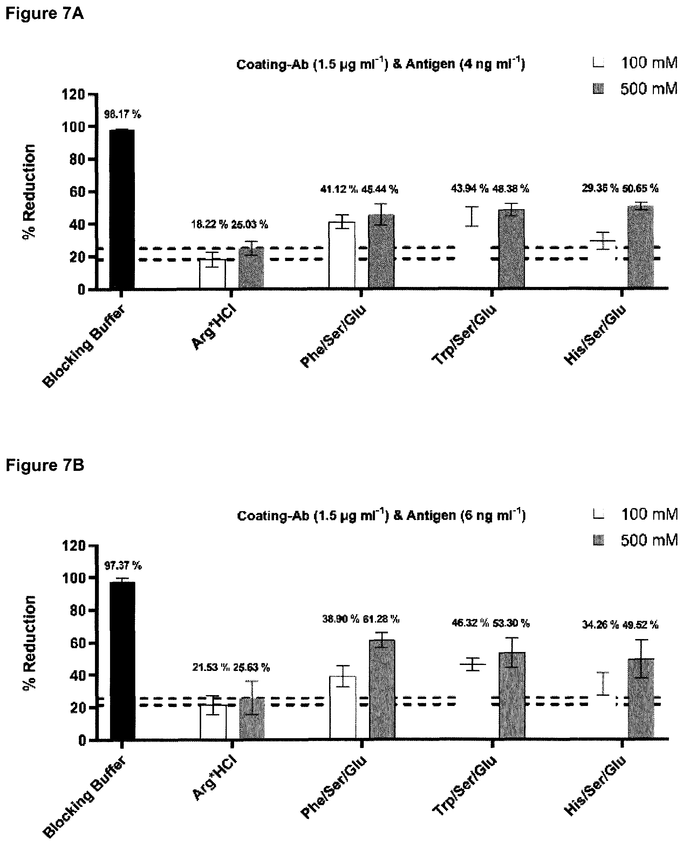

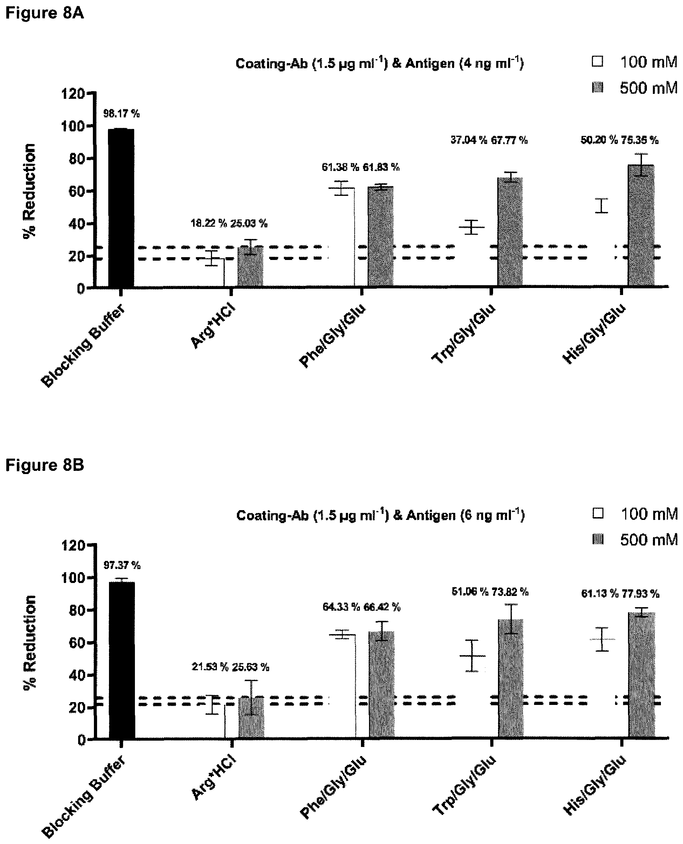

As shown in Example 1.7, Figures 7 and 8, the inventors surprisingly found that the addition of an amino acid selected from either group (b) an amino acid with a polar uncharged R or an amino acid from group (a) an amino acid with an unpolar aliphatic R group to a composition comprising two amino acids from each of groups (d) and (e) significantly increased the reduction of surface adsorption as compared to a composition comprising only two amino acids from groups (d) and (e).

Thus, in a preferred embodiment of the invention the composition comprises at least three different amino acids wherein the at least three amino acids are at least one amino acid selected from group (b) of amino acids with a polar uncharged R, or at least one amino acid from group (a) of amino acids with an unpolar aliphatic R group, and further at least one amino acid selected from group (d) of amino acids with negatively charged R groups; and at least one amino acid selected from group (e) of amino acids with aromatic R groups. The composition may also comprise no other amino acids than the three amino acids as described afore. More preferably the amino acid selected group (a) is glycine, the amino acid selected from group (b) is serine, the amino acid selected group (d) is glutamine and the amino acid selected from group (e) is phenylalanine, tryptophane or histidine.

In a specific embodiment, the composition may not comprise arginine and/or lysine other than in the polypeptide.

The skilled person further understands that it is not necessary that the same number of amino acids of each group is present in the composition used according to the invention. Rather, any combination of amino acids can be chosen as long as at least one amino acids of each group is present.

The composition comprises a total amino acid concentration from 10 mM to 550 mM, such as from 10 mM to 500 mM, from 10 mM to 400 mM, from 10 mM to 300 mM, from 10 mM to 250 mM, from 10 mM to 160 mM, from 10 mM to 140 mM, or from 10 mM to 120 mM

In a preferred embodiment the composition may comprise at least one amino acid selected from group (a) and at least one amino acid selected from group (c) in a total amino acid concentration of up to 150 mM, of up to 150 mM, about 50 mM to about 175 mM, about 70 mM to about 150 mM, or about 70 mM to about 120 mM. The amino acid selected from group (a) may be in excess over amino acid selected group (c). Preferably the excess in concentration ratio of amino acid selected group (a) to amino acid selected from group (c) may be at least 1,5 to 1; at least 2 to 1, or at least 2,5 to 1.

In a preferred embodiment the composition may comprise at least one amino acid selected from group (c) and at least one amino acid selected from group (e) in a total amino acid concentration of up to 150 mM, of up to 150 mM, about 50 mM to about 175 mM, about 70 mM to about 150 mM, or about 70 mM to about 120 mM. In one embodiment the amino acid selected from group (c) may be in excess over amino acid selected group (e) or the amino acids may be present in equal amounts.

In a preferred embodiment the composition may comprise at least one amino acid selected from group (a) and at least one amino acid selected from group (e) of amino acids in a total amino acid concentration of up to 150 mM, of up to 150 mM, about 50 mM to about 175 mM, about 70 mM to about 150 mM, or about 70 mM to about 120 mM.

In a preferred embodiment the composition may comprise at least one amino acid selected from group (b) and at least one amino acid selected from group (e) of amino acids in a total amino acid concentration of up to 150 mM, of up to 150 mM, about 50 mM to about 175 mM, about 70 mM to about 150 mM, or about 70 mM to about 120 mM.

Furthermore, the amino acids can be present in the composition as singular molecules and/or as di- and/or tripeptides, preferably singular molecules.

In another preferred embodiment of the invention, one or more of the amino acids are selected from the group consisting of natural non-proteinogenic and synthetic amino acids.

The term "non-proteinogenic amino acids", in accordance with the present invention, relates to amino acids that are not naturally incorporated into polypeptides and proteins. Non-proteinogenic amino acids can be derived from proteinogenic amino acids, which are La-amino acids, by post-translational modifications. Such non-proteinogenic amino acids are, for example, lanthionine, 2-aminoisobutyric acid, dehydroalanine, and the neurotransmitter gamma-aminobutyric acid. Also the D-enantiomers of proteinogenic L-amino acids represent non-proteinogenic amino acids. Further non-limiting examples of non-proteinogenic amino acids include carnitine, creatine, creatinine, guanidinoacetic acid, ornithine, hydroxyproline, homocysteine, citrulline, hydroxylysine or beta-alanine.

The term "synthetic amino acids", as used herein, relates to amino acids not naturally occurring in nature. Non-limiting examples of synthetic amino acids include (2R)-amino-5-phosphonovaleric acid, D-phenyl glycine or (S)- and (R)-tert-leucine.

The composition comprising the polypeptide and at least two different amino acids may be a solid, liquid or frozen composition. A solid composition will usually be solubilized in a liquid before administering the polypeptide to a subject. Typically, a solid composition will be a dried, preferably freeze- or spray dried composition. A liquid composition according is preferably an aqueous composition. A frozen composition will usually be thawed to obtain a liquid composition before administering the polypeptide to a subject.

In a further embodiment, composition of the method of the invention further comprises at least one sugar. In an embodiment of the invention, the composition comprises an amino acid-sugar ratio of at least 1:2.

The term "sugar", as used herein, refers to any types of sugars, i.e. the monosaccharide, disaccharide or oligosaccharide forms of carbohydrates as well as sugar alcohols. Examples of suitable sugars include, without being limiting, trehalose, saccharose, sucrose, glucose, lactose, mannitol, and sorbitol or sugar derivatives such as amino sugars, e.g. glucosamine or n-acetyl glucosamine.

Preferred amounts of sugars to be comprised in the composition according to the invention are between 0.1 mg/ml to 200 mg/ml sugar, more preferably between 10 mg/ml to 180 mg/ml sugar, even more preferably between 20 mg/ml to 160 mg/ml sugar and most preferably the amount is about 80 mg/ml sugar. Where a mixture of different types of sugars is employed, these preferred amounts refer to the sum of all sugars in the composition.

Preferably, the composition comprises trehalose or sucrose as the sugar and mannitol as the sugar alcohol.

Furthermore, the composition is characterized by an amino acid to sugar ratio of at least 1:2. More preferably, the composition is characterized by an amino acid to sugar ratio of at least 1:1.5 (w/w), such as e.g. at least 1:1 (w/w) and most preferably of at least 1:0.1(w/w).

In another embodiment, the composition may be substantially free of sugar. The term "free or substantially free of (a) sugar", in accordance with the present invention, refers to a composition devoid of or substantially devoid of any types of sugars, i.e. the monosaccharide, disaccharide or oligosaccharide forms of carbohydrates as well as sugar alcohols. Examples of sugars commonly used in polypeptide formulations, but absent in accordance with the present invention, include without being limiting saccharose, trehalose, sucrose, glucose, lactose, sorbitol or mannitol. The composition is considered to be substantially free of sugar if it contains less than 0.1% (w/v) sugar, more preferably less than 0.01% (w/v) and even more preferably less than 0.001% (w/v) and most preferably less than 0.0001% (w/v).

Advantageously, reduction of adsorption of a polypeptide on a surface according to the present invention by using amino acid-based compositions reduces or obviates the need for adding additional stabilizing proteins to the composition to inhibit adsorption of the polypeptide of interest on a surface. Therefore, the composition according to the invention may be free or substantially free of at least one stabilising protein.

In a further embodiment of the invention the surface as not been subjected to a composition comprising stabilising protein or mixtures of amino acids, generally known as blocking compositions, prior to being in contact with a composition according to the invention comprising a polypeptide and at least two different amino acids as described herein. Among other compositions comprising stabilising protein or mixtures of amino acids, these compositions include for example cell culture media, calf or other animal sera, solutions of milk powder and commonly used solutions of multiple amino acids for infusions, e.g. Glamin (Baxter).

The term "stabilising protein" refers to proteins other than the polypeptide which adsorption is prevented according to the present invention. As described herein the "polypeptide" is preferably a therapeutic protein, such as a therapeutic antibody, whereas the "stabilising protein" is preferably a protein having no therapeutic activity. Preferably the stabilising protein is an albumin, most preferably human serum albumin (HSA) or bovine serum albumin (BSA).

As used herein, the term "free or substantially free of at least one stabilising protein" refers to a composition that does not comprise or does substantially not comprise any protein(s) other than the polypeptide whose. It will be appreciated by the skilled person that trace amounts of proteins associated with e.g. contamination of the composition may be present and are not excluded by the requirement that the composition is free of at least one stabilising protein. Thus, the composition is considered to be substantially free of at least one stabilising protein if it contains less than 0.1% (w/v) proteins other than the polypeptide which, more preferably less than 0.01% (w/v) and even more preferably less than 0.001 % (w/v) and most preferably less than 0.0001 % (w/v).

In an alternative embodiment, the composition may comprise at least one stabilizing protein to further increase the anti-adsorption effect of the invention.

Further to the different excipients described above, the composition may comprise additional excipients. Such additional excipients are preferably selected from chelating agents, additional anti-oxidative agents and surfactants.

The term "chelating agents", as used herein, relates to excipients that trap metal ions in formulations to avoid e.g. metal ion-catalysed oxidative reactions within a formulation. Non-limiting examples of chelating agents include desferal, diethyltriaminepentaactic acid (DTPA), ethylenediaminetetraacetic acid (EDTA), or deferoxamine (DFO). Whereas such chelating agents are commonly used in chelation therapy to detoxify poisonous metal agents such as mercury [Hg], arsenic [As], and lead [Pb] by converting them to a chemically inert form that can be excreted without further interaction with the body, it will be understood that the chelating agents are used in accordance with the present invention in low concentrations, e.g. between 0.3 and 0.5 mg/ml, that will not elicit a therapeutic effect, but rather stabilize the biopharmaceutical products during e.g. storage.

The term "additional anti-oxidative agents", as used herein, relates to methionine, cysteine, glutathione, tryptophan, histidine, ascorbic acid and any derivatives of the herein listed agents, without being limiting.

Advantageously, reduction of adsorption of a polypeptide on a surface according to the present invention by using amino acid-based compositions obviates or reduces the need for adding surfactants, for example polysorbates. Accordingly, the composition of the present invention may only comprise less than 0.01% (w/v), preferably less than 0.001 % (w/v), more preferably less than 0.0001 % (w/v), most preferably less than 0.00001% (w/v) surfactant. In a specific embodiment, the composition is free or substantially free of at least one surfactant.

The term "surfactant", as used herein, relates to surface-active agents. This term also includes wetting agents, emulsifying agents and suspending agents, depending on their properties and use. Surface-active agents are substances which, at low concentrations, adsorb onto the surfaces or interfaces of a system and alter the surface or interfacial free energy and the surface or interfacial tension. Because they are soluble in both organic solvents and water, they are called "amphiphilic". Preferred surfactants in accordance with the present invention include non-ionic surfactants. More preferably, the surfactants according to the invention are polysorbates, especially polysorbate 20 (Tween 20) and polysorbate 80 (Tween 80), triblock copolymers composed of a central hydrophobic chain of polyoxypropylene (poly(propylene oxide)) flanked by two hydrophilic chains of polyoxyethylene (poly(ethylene oxide), for example Synperonics^{®}, Pluronics^{®}, and Kolliphor^{®}.

In an alternative embodiment, the composition may comprise at least one surfactant, for example a surfactant selected from the surfactants described afore, to further increase the anti-adsorption effect according of the method of the invention. The composition may comprise at least 0.01% (w/v), preferably less than 0.05% (w/v), more preferably at least 0.1% (w/v).

The composition may further comprise salts, for example monovalent or divalent salts, such as sodium chloride, potassium chloride, magnesium chloride, calcium chloride; or sulphate, carbonate or acetate salts of the afore mentioned cations. The salts may for example be comprised in a concentration of up to 100 mM, up to 50 mM, or up to 25 mM.

As described above, in compositions with a low concentration of therapeutic biomolecules, the ratio of protein adsorbed to a surface to solubilized protein may be particularly high. Thus, in a preferred embodiment, the composition comprising a polypeptide according to the present invention is characterized by a low polypeptide concentration not higher than 20 mg/ml, 10 mg/ml, 5 mg/ml, or 1 mg/ml. Preferably polypeptide concentration is not higher than 10 mg/ml, more preferably not higher than 5 mg/ml, most preferably not higher than 2 mg/ml.

As described above, highly concentrated biomolecules are susceptible to aggregation induced by surface interaction. Thus, in an alternative preferred embodiment the composition comprising a polypeptide according to the present invention is characterized by a high polypeptide concentration. The polypeptide concentration may for example be at least 80 mg/ml, at least 100 mg/ml, at least 120 mg/ml, at least 150 mg/ml, at least 180 mg/ml, at least 200 mg/ml, or at least 250 mg/ml. Preferably the polypeptide concentration is at least 100 mg/ml, more preferably at least 150 mg/ml, and most preferably at least 200 mg/ml.

The surface according to the present invention comprises or consists of a metal, a glass or a polymer material. Preferably the surface may be a polymer material. Preferably the polymer material is selected from polyethylene (PE), for example selected from polyethylene terephthalate (PET), high density polyethylene (HDPE), low-density polyethylene (LDPE), linear low density polyethylene (LLDPE), polypropylene (PP), polystyrene (PS), polyvinylchloride (PVC), polyvinylidene chloride (PVDC), polytetrafluorethylene (PTFE), polyethersulphone (PES), polymethylmethacrylate, polycarbonate (PC, bisphenol A), nylon, polyether urethane, polysiloxane (silicone), polychlorotrifluoroethylene (PCTFE)/PVC laminates, polyamide (PA), e.g. orientated polyamide (OPA), cyclic olefin copolymer, or mixtures and copolymers thereof. The polymer may further be natural or synthetic elastomer such as for example latex, polyisoprene rubber, chloroprene (2-chloro-1,3-butadiene), styrene-butadiene rubbers (SBR) silicone rubber, and butyl rubber.

In a preferred embodiment, the polymer material is a hydrophobic polymer material. More preferably the polymer material is selected from polypropylene (PP), polystyrene (PS), or polyvinylchloride (PVC).

In another preferred embodiment the polymer material is a polymer material comprising aromatic groups, for example polystyrene.

The surface may also be a metal or glass material coated with the above-mentioned polymers.

As hydrophobic surfaces interact with unfolded polypeptide stretches and may thus induce adsorption and aggregation of polypeptides, the surface according to the invention is preferably a hydrophobic surface.

In a preferred embodiment, the surface is part of a medical device.

Accordingly, the invention also relates to a medical device comprising a composition as described above comprising the polypeptide and at least two different amino acids. In said medical device, the polypeptide is exposed to a surface of the medical device.

The term "medical device" as used herein includes articles of manufacture that are used in treating a patient and/or administering a therapeutic polypeptide to a patient. A medical device can include articles of manufacture such as syringes and syringe assemblies, drug cartridges, metering dispensers for therapeutic liquids, tubes and valves for therapeutic liquids, catheters, shunts, tubing needles, needleless injectors, implantable devices, pumps especially osmotic pumps, infusion bags, vials and other types of containers preferably used during the administration of a polypeptide to a patient, and the like.

### Figure Legends

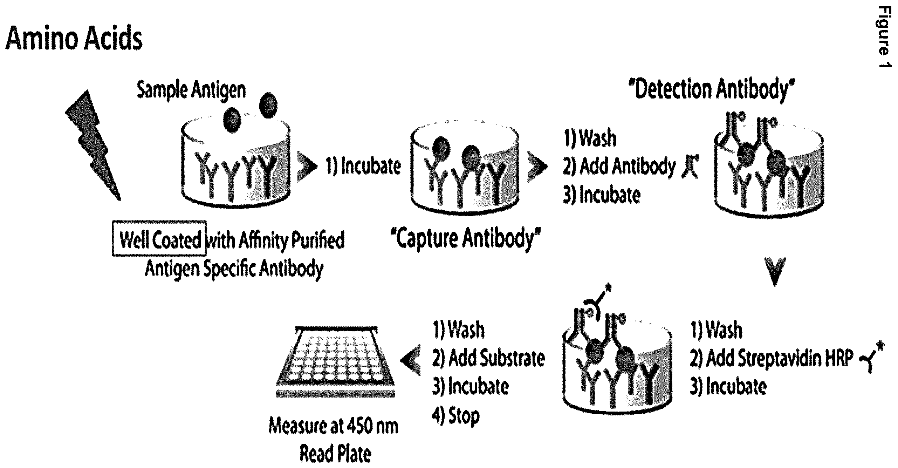

**Figure 1** shows a schematic representation of the work flow of an ELISA experiment in a well of a 96 well plate. The protein adsorption reducing formulations (Amino Acids; red arrow) interfere with the coating of the wells using the anti-IL6-antibody (antigene specific antibody) diluted in these amino acid containing formulations.

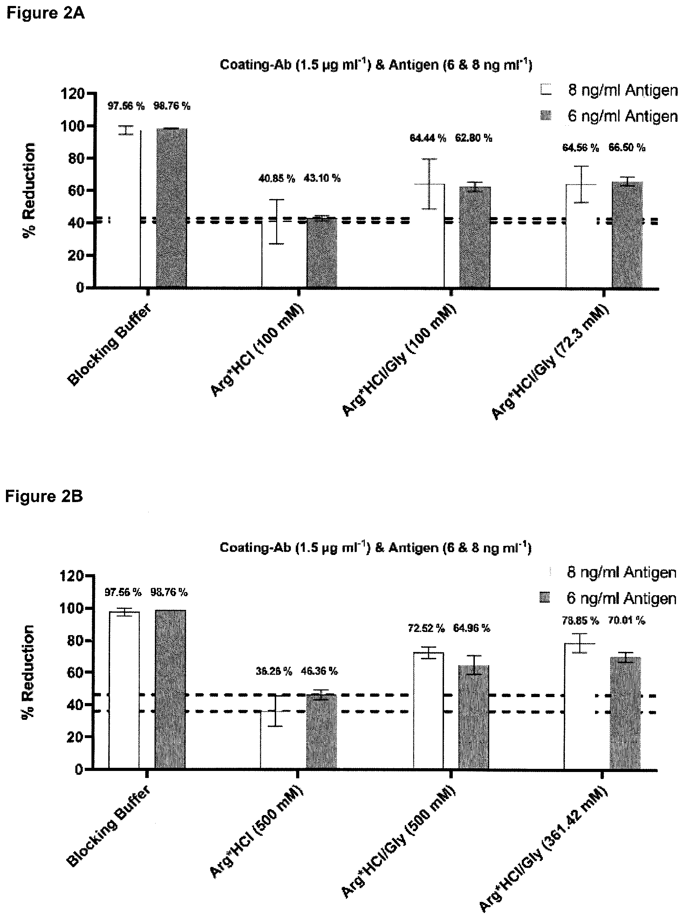

**Figure 2** shows the reduction of adsorption of an anti-IL6 coating antibody onto a polystyrene medium binding surface in the presence of (A) 100 mM Arg*HCl; a combination of Arg*HCl/Glycine with a total concentration of 100 mM and a combination of Arg*HCl/Glycine with a total concentration of 72.3 mM and (B) 500 mM Arg*HCl; a combination of Arg*HCl/Glycine with a total concentration of 500 mM and a combination of Arg*HCl/Glycine with a total concentration of 361.42 mM. The positive control representing the maximum reduction of protein surface adsorption (100 %) is depicted as Blocking Buffer and the negative control representing no reduction of protein surface adsorption was the anti-IL6-antibody diluted in PBS. The concentration of the anti-I L6-antibody was 1.5 µg/ml and the IL6 antigen concentrations were 6 ng/ml (gray bars) and 8 ng/ml (white bars). The dashed lines represent the % reduction of the surface adsorption in the presence of Arg*HCl at 100 mM and 500 mM respectively. The quantities for the % reduction of the adsorption of the anti-IL6 coating antibody are depicted on top of the bars in %.

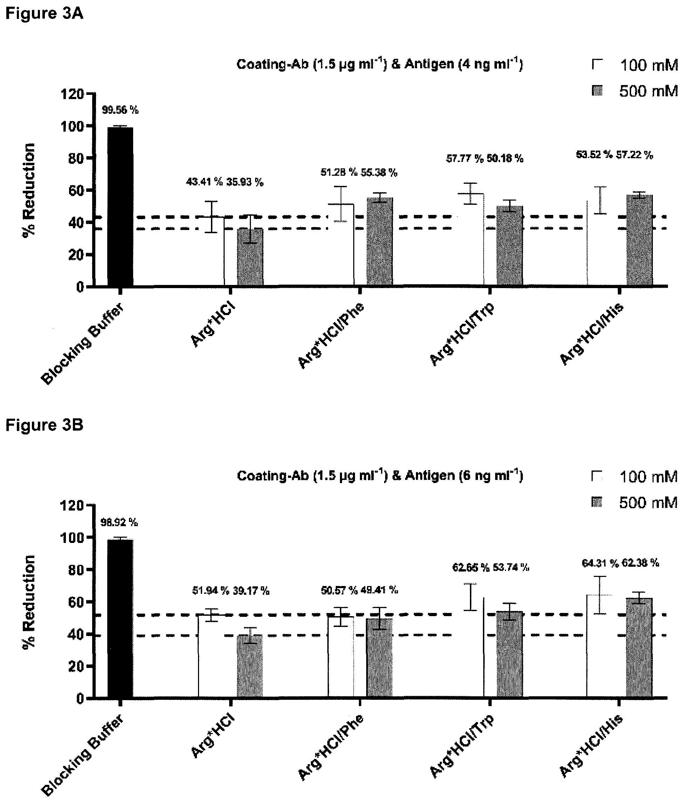

**Figure 3** shows the reduction of adsorption of an anti-IL6 coating antibody onto a polystyrene medium binding surface in the presence of 100 mM Arg*HCl (white bars) as well as 500 mM Arg*HCl (gray bars) and Arg*HCl in combination with aromatic amino acids phenylalanine, tryptophan and histidine at total concentrations of 100 mM (white bars) and 500 mM (gray bars), respectively. The concentration of the coating anti-IL6-antibody was 1.5 µg/ml and the IL6 antigen concentration was 4 ng/ml in (A) and 6 ng/ml in (B). The dashed lines represent the % reduction of the surface adsorption in the presence of Arg*HCl. The positive control for maximal reduction of anti-IL6 coating antibody adsorption (approx. 100 %) is Blocking Buffer (black bars). The quantities for the % reduction of the adsorption of the anti-IL6 coating antibody are depicted on top of the bars in %.

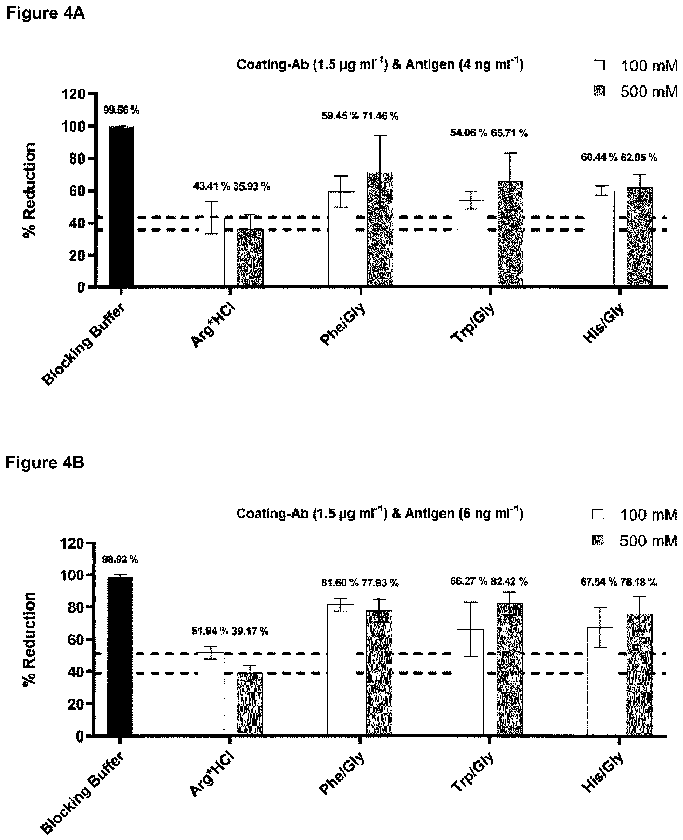

**Figure 4** shows the reduction of adsorption of an anti-IL6 L6 coating antibody onto a polystyrene medium binding surface in the presence of 100 mM Arg*HCl (white bars) as well as 500 mM Arg*HCl (gray bars) and combinations of the aromatic amino acids phenylalanine, tryptophan and histidine with non-polar amino acid glycine at total concentrations of 100 mM (white bars) and 500 mM (gray bars), respectively. The concentration of the coating anti-IL6-antibody was 1.5 µg/ml and the IL6 antigen concentration was 4 ng/ml in (A) and 6 ng/ml in (B). The dashed lines represent the % reduction of the surface adsorption in the presence of Arg*HCl. The positive control for maximal reduction of anti-IL6 coating antibody adsorption (approx. 100 %) is Blocking Buffer (black bars). The quantities for the % reduction of the adsorption of the anti-IL6 coating antibody are depicted on top of the bars in %.

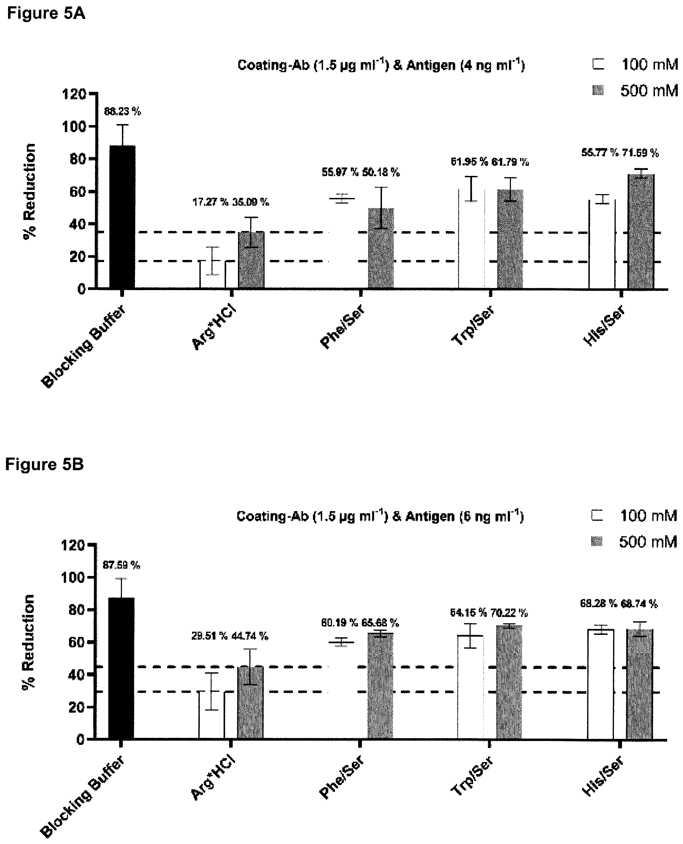

**Figure 5** shows the reduction of adsorption of an anti-IL6 coating antibody onto a polystyrene medium binding surface in the presence of 100 mM Arg*HCl (white bars) as well as 500 mM Arg*HCl (gray bars)and combinations of the aromatic amino acids phenylalanine, tryptophan and histidine with the polar amino acid serine at total concentrations of 100 mM (white bars) and 500 mM (gray bars), respectively. The concentration of the coating anti-IL6-antibody was 1.5 µg/ml and the IL6 antigen concentration was 4 ng/ml in (A) and 6 ng/ml in (B). The dashed lines represent the % reduction of the surface adsorption in the presence of Arg*HCl. The positive control for maximal reduction of anti-IL6 coating antibody adsorption (approx. 100 %) is Blocking Buffer (black bars). The quantities for the % reduction of the adsorption of the anti-IL6 coating antibody are depicted on top of the bars in %.

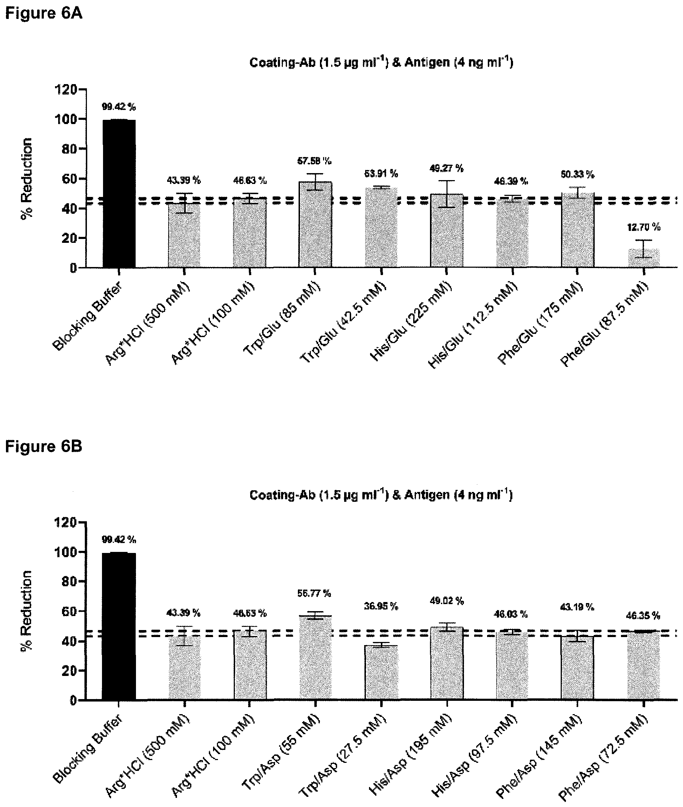

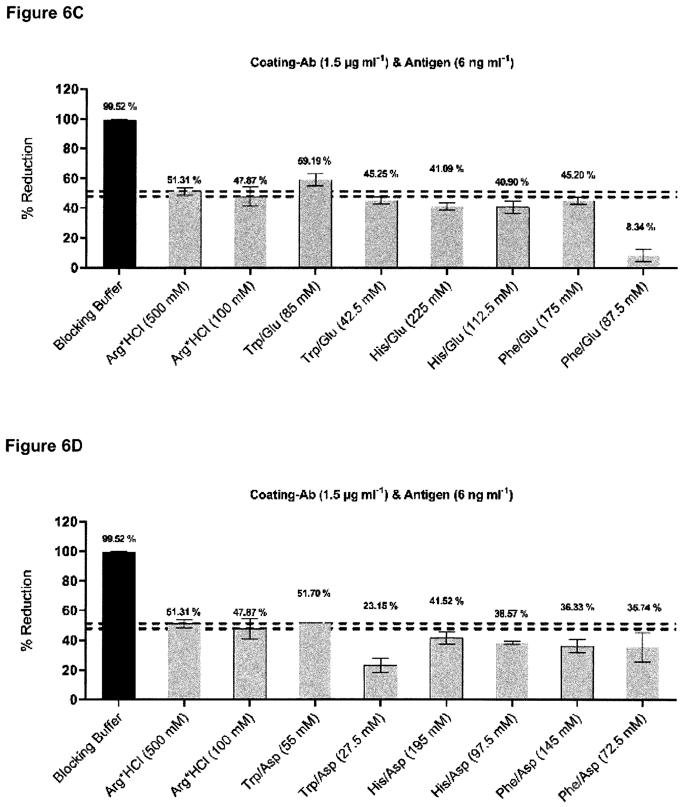

**Figure 6** shows the reduction of adsorption of an anti-IL6 coating antibody onto a polystyrene medium binding surface in the presence of 500 mM Arg*HCl as well as 100 mM Arg*HCl and combinations of the aromatic amino acids phenylalanine, tryptophan and histidine with the negatively charged amino acid glutamic acid (A and C) and aspartic acid (B and D) at different total concentrations remarkably smaller than 500 mM and 100 mM. The concentration of the coating anti-IL6-antibody was 1.5 µg/ml and the IL6 antigen concentration was 4 ng/ml in (A) as well as (B) and 6 ng/ml in (C) as well as (D). The dashed lines represent the % reduction of the surface adsorption in the presence of 500 mM and 100 mM Arg*HCl.
**Figure 7** shows the reduction of adsorption of an anti-IL6 coating antibody onto a polystyrene medium binding surface in the presence of 100 mM Arg*HCl (white bars) as well as 500 mM Arg*HCl (gray bars) and combinations of the aromatic amino acids phenylalanine, tryptophan and histidine with the polar amino acid serine and the negatively charged amino acid glutamic acid at total concentrations of 100 mM (white bars) and 500 mM (black bars), respectively. The concentration of the coating anti-IL6-antibody was 1.5 µg/ml and the IL6 antigen concentration was 4 ng/ml in (A) and 6 ng/ml in (B). The dashed lines represent the % reduction of the surface adsorption in the presence of Arg*HCl. The positive control for maximal reduction of anti-IL6 coating antibody adsorption (approx. 100 %) is Blocking Buffer (black bars). The quantities for the % reduction of the adsorption of the anti-IL6 coating antibody are depicted on top of the bars in %.
**Figure 8** shows the reduction of adsorption of an anti-IL6 coating antibody onto a polystyrene medium binding surface in the presence of 100 mM Arg*HCl (white bars) as well as 500 mM Arg*HCl (gray bars) and combinations of the aromatic amino acids phenylalanine, tryptophan and histidine with the non-polar amino acid glycine and the negatively charged amino acid glutamic acid at total concentrations of 100 mM (white bars) and 500 mM (gray bars), respectively. The concentration of the coating anti-IL6-antibody was 1.5 µg/ml and the IL6 antigen concentration was 4 ng/ml in (A) and 6 ng/ml in (B). The dashed lines represent the % reduction of the surface adsorption in the presence of Arg*HCl. The positive control for maximal reduction of anti-IL6 coating antibody adsorption (approx. 100 %) is Blocking Buffer (black bars). The quantities for the % reduction of the adsorption of the anti-IL6 coating antibody are depicted on top of the bars in %.

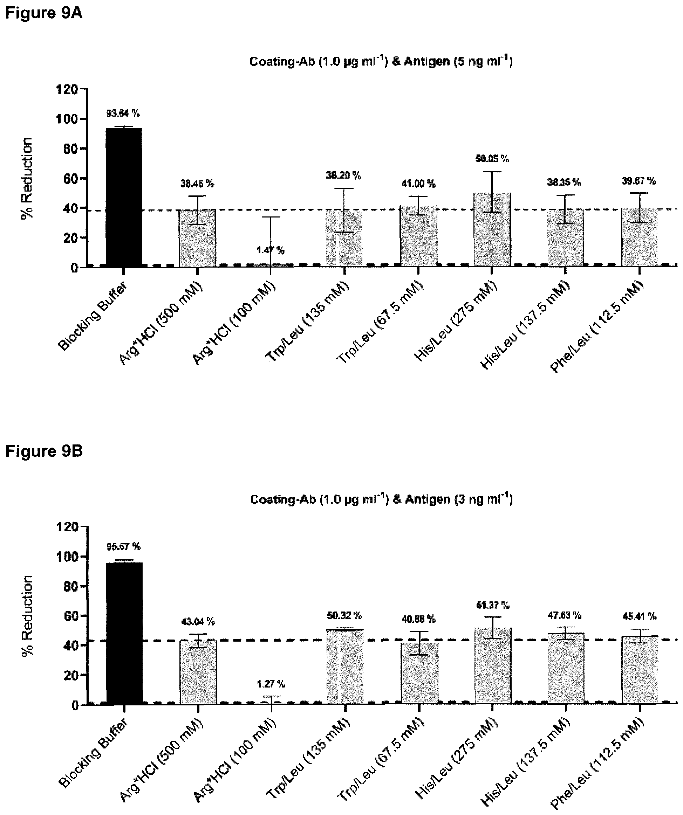

**Figure 9** shows the reduction of adsorption of an anti-IL6 coating antibody onto a hydrophobic polypropylene surface in the presence of 100 mM Arg*HCl and 500 mM Arg*HCl and combinations of the aromatic amino acids phenylalanine, tryptophan and histidine with the non-polar, hydrophobic, branched amino acid leucine at different total concentrations remarkably smaller than 100 mM and 500 mM. The concentration of the coating anti-IL6-antibody was 1 µg/ml and the IL6 antigen concentration was 3 ng/ml in (A) and 5 ng/ml in (B). The dashed lines represent the % reduction of the surface adsorption in the presence of 100 mM and 500 mM Arg*HCl.

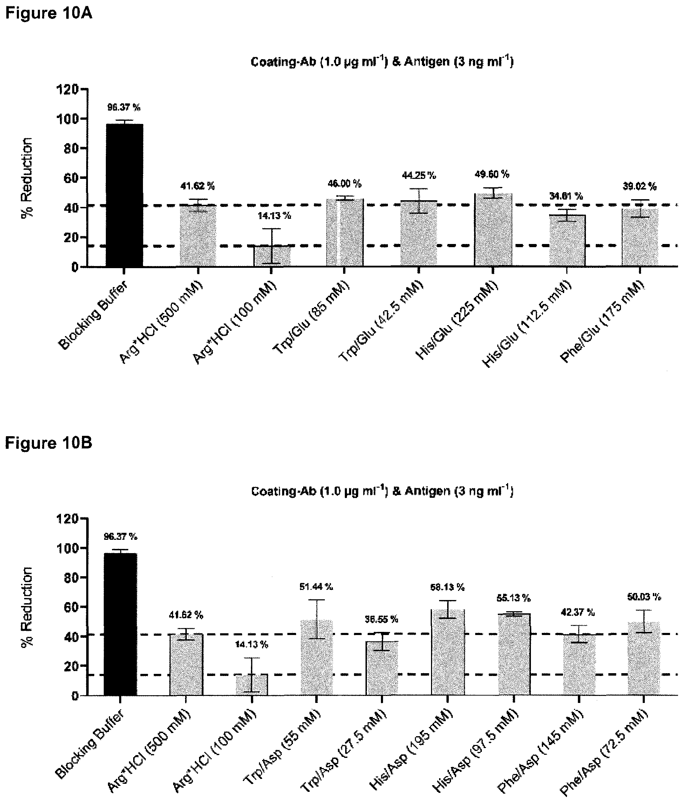

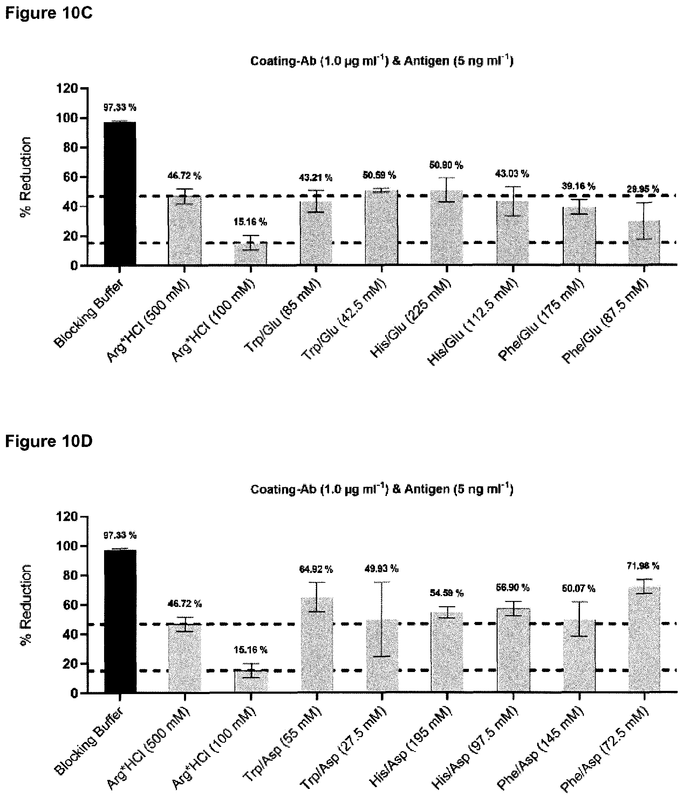

**Figure 10****:** See example 1.9.

The following examples exemplify the invention described afore.

### Example 1: Surface Absorption of anti-IL-6 antibody

### 1.1. Materials and Methods:

In order to directly analyze the reduction of surface adsorption of biomolecules particularly of proteins on solid surfaces by different amino acid mixtures an anti-IL6-ELISA was applied as a model for characterization and quantification of surface adsorption of an anti-IL6 coating antibody (IgG1) as modell protein. ELISA technology is a well known surface-sensitive technique to characterize the adsorption of biomolecules on surfaces with a high specificity and sensitivity and the possibility for automatization. ELISA is a technique that uses solid supports surfaces (multi-well plates or polystyrene beads) for the molecular quantification of immune-complexes via signal amplification through an enzymatic reaction readout. The reaction rates in all steps are limited by diffusion of the biomolecules generating therefore relatively long incubation times (h).

In the ELISA assay used to analyse the reduction of surface adsorption according to the invention, adsorption of an anti-IL6 coating antibody on a hydrophobic polystyrene (PS) or polypropylene (PP) medium binding 96 well plate was directly quantified in the presence and absence of solutions in accordance with the liquid compositions of invention comprising and at least two different amino acids during coating of the wells with anti-IL6. The anti-IL6 antibody was diluted in different liquid composition prior the coating step. As comparison, the surface adsorption in the solutions according to the invention was compared to adsorption in 100 mM and 500 mM arginine*HCl as disclosed by Shikiya et al. (Shikiya Y,Tomita S, Arakawa T, Shiraki K. Arginine inhibits adsorption of proteins on polystyrenesurface (2013). PLoS One, 8 (8), e70762).

For quantification of the relative reduction of surface adsorption of the anti-IL6 coating antibody, the photometric absorption of positive reference samples using Blocking Buffer for dilution and incubation was defined as 100% reduction of adsorption and the photometric absorption of negative reference samples using PBS buffer or a low ionic strength 10 mM sodium phosphate buffer at pH 7.4 without any other additives for dilution and incubation was defined as 0% reduction of adsorption.

In detail, after dilution of the anti-IL6-antibody (monoclonal mouse IgG1; R&D systems Inc., USA) with PBS, Blocking Buffer (0,05 % Polysorbat 20, 0,01 % skim milk powder in PBS pH 7.4), solutions comprising arginine*HCl, or solutions comprising at least two amino acids according to the invention, coating of the antibody in the respective solutions was perfomred at 2 - 8 °C for 17 hours over night on polystyrene midium binding plates (Greiner Bio One^{™} 96-well Microtiter plates, Germany) or polypropylene plates (Eppendorf, Germany) at a anti-IL6 concentration of 1.5.µg/ml. After removal anti-IL6-antibody of solutions, the wells were incubated . for one hour at room temperature with Blockin Buffer 2 comprising 5 % skim milk powder (Sigma Aldrich, Germany) in 0,05 % Polysorbat 20, 0,01 % skim milk powder in PBS pH 7.4 to block the free binding sites of the well surface. Subsequently the wells were incubated for two hours at room temperature with the recombinant human anti-IL6-antigen (R&D systems Inc., USA) at concentrations of 4 and ng/ml and for additional 1 hour with the biotinylated human anti-IL6 detection antibody (R&D systems Inc., USA). Afterwards the wells were incubated for 20 min at room temperature in the dark with Streptavidine-HRP conjugate (R&D systems Inc., USA). For the enzymatic reaction the TMB and H₂O₂ substrate solution (Thermo Fisher Scientific, Germany) was added and incubated for further 20 min at 37 °C. After stopping the enzymatic conversion of 3, 3', 5, 5'-Tetramethylbenzidine TMB with 3.6 M H₂SO₄ leading to a color change from blue to yellow, the yellow product was detected photometrically at 450 nm using a plate reader. The procedure is schematically depicted in Figure 1.

Finally, the calculation of the reduction of surface adsorption based on the photometric absorption of the samples was performed as explained above.

Example 1.2: Reduction of the surface adsorption on polystyrene in the presence of liquid compositions comprising the positively charged amino acid arginine and arginine in combination with the non-polar; osmolytic amino acid glycine at different total amino acid concentrations.

In the first example, the surface adsorption in solutions comprising arginine and combinations of arginine with glycine at total amino acid concentrations of 72.3 mM, 100 mM, 361.42 mM and 500 mM in water was analysed as follows:
Arg*HCl (500 mM) without other additives
Arg*HCl (100 mM) without other additives
Arg*HCl/Gly (500 mM): 130 mM Arg*HCl + 370 mM Gly (Argininge to Glycine 1/2.8)
Arg*HCl/Gly (361.42 mM): 95 mM Arg*HCl + 266.42 mM Gly (Argininge to Glycine 1/2.8)
Arg*HCl/Gly (100 mM): 29 mM Arg*HCl + 71 mM Gly (Argininge to Glycine 1/2.8)
Arg*HCl/Gly (72.3 mM): 19 mM Arg*HCl + 53.3 mM Gly (Argininge to Glycine 1/2.8)

Surface adsorption was analysed in the anti-IL6-ELISA as described in example 1.1. As shown in Figure 2, surface adsorption of the coating anti-IL6-antibody in the presence of 100 mM and 500 mM of the positively charged amino acid arginine alone was reduced to values between 36 % and 46 %.

The addition of the non-polar, osmolytic amino acid glycine to the positively charged amino acid arginine in a molar excess and a concentration ratio of 2.8 : 1 (non-equimolar) resulted in a remarkable increase of reduction of the anti-IL6-antibody adsorption on the polystyrene medium binding plates to 60 % - 80 %. Even the combination of the positively charged amino acid arginine with the non-polar amino acid glycine in total amino acid concentrations of 72.3 mM and 361.42 mM smaller than the arginine concentrations of 100 and 500 mM and the concomitant reduction of the corresponding arginine concentration to 19 mM and 95 mM resulted in a similar reduction of the surface adsorption of the anti-IL6-antibody to the polystyrene medium binding plate.

The observed reduction in surface adsoption indiacates that the addition of a molar excess of glycine to smaller concentrations of arginine remarkably increased the amount of reduction of protein adsorption in comparison to higher molar concentrations of arginine alone.

Example 1.3: Reduction of the surface adsorption on polystyrene in the presence of liquid compositions comprising the positively charged amino acid arginine in combination with aromatic amino acids phenylalanine, tryptophan and histidine.

To exclude effects related to other buffer components in comparison to pure water used in the amino acid solutions in example 1. 2 amino acid solution in this example and the following examples were prepared in PBS at pH 7.4. Due to the limited solubility of aromatic amino acids, aromatic amino acids could not be used in equimolar concentration in relation to arginine in the respective solutions. The following solutions were analysed for surface adsorption in the anti-IL6-ELISA as described in example 1.1.:
Arg*HCl (500 mM) without other additives
Arg*HCl (100 mM) without other additives
Arg*HCl/Phe (500 mM)= 375 mM Arg*HCl + 125 mM Phe
Arg*HCl/Phe (100 mM)= 50 mM Arg*HCl + 50 mM Phe
Arg*HCl/Trp (500 mM)= 465 mM Arg*HCl + 35 mM Trp
Arg*HCl/Trp (100 mM)= 75 mM Arg*HCl + 25 mM Trp
Arg*HCl/His (500 mM)= 325 mM Arg*HCl + 175 mM His
Arg*HCl/His (100 mM)= 50 mM Arg*HCl + 50 mM His

As shown in Figure, a remarkably stronger reduction of the surface adsorption of the anti-IL6 coating antibody in the presence of the non-equimolar mixtures of the positively charged amino acid arginine and the aromatic amino acids in comparison to arginine alone was observed. The addition of the aromatic amino acids to the molar concentration excess of the positively charged amino acid arginine resulted in the remarkable increase of the reduction of the % surface adsorption, particularly at the lower antigen concentration of 4 ng/mL (55.38 % arginine/phenylalanine; 55.18 and 57.77 % % arginine/tryptophan; 57.22 % arginine/histidine). In the case of the higher IL6-antigen concentration of 6 ng/mL the % reduction of the surface adsorption in the presence of equimolar concentrations of phenylalanine is comparable to arginine alone at a concentration of 100 mM (50.57 % arginine/phenylalanine versus 51.94 % arginine alone). Surprisingly, although arginine is present all mixtures at a molar excess at total amino acid concentration of 500 mM, the % reduction of the surface adsorption of the coating antibody is higher than in the presence of arginine alone at a concentration of 500 mM. For example, at the higher antigen concentration of 6 ng/mL the observed adsorption reduction was observed for arginine/phenylalanine at a total amino acid concentration of 500 mM to be 49.41 % versus 39.17 % arginine 500 mM alone. The addition of the aromatic amino acids tryptophan and histidine to arginine in a molar excess resulted in a stronger reduction of the surface adsorption compared to phenylalanine in combination with arginine in both concentration ranges (Figure 3). The effectivity of the aromatic amino acids in combination with the positively charged basic amino acid arginine increases in the order phenylalanine < tryptophan < histidine.

In the presence of 100 mM of the positively charged amino acid arginine alone, the % reduction of the protein adsorption was around 43 - 52 %. In the presence of 500 mM arginine, the reduction of the surface adsorption of the anti-IL6-antibody was around 36 - 40 %. It should be noted, that the concentrations of the liquid compositions containing arginine alone were in each case higher than the arginine concentrations in the mixtures. So, the increasing effectivities of the liquid compositions containing a molar excess of arginine in combination with the aromatic amino acids against protein surface adsorption are clearly the result of the addition of the aromatic amino acids to the molar excess concentration of arginine.

Example 1.4: Reduction of the surface adsorption on polystyrene in the presence of liquid compositions comprising combinations of the aromatic amino acids phenylalanine, tryptophan and histidine with the non-polar amino acid glycine without arginine.

The following solutions amino acid solutions in PBS were analysed for surface adsorption in the anti-IL6-ELISA as described in example 1.1.:
Arg*HCl (500 mM) without other additives
Arg*HCl (100 mM) without other additives
Phe/Gly (500 mM) = 125 mM Phe + 375 mM Gly
Phe/Gly (100 mM) = 50 mM Phe + 50 mM Gly
Trp/Gly (500 mM) = 35 mM Trp + 465 mM Gly
Trp/Gly (100 mM) = 25 mM Trp + 75 mM Gly
His/Gly (500 mM) = 175 mM His + 325 mM Gly
His/Gly (100 mM) = 50 mM His + 50 mM Gly

As shown in Figure 4, the combination of aromatic amino acids (phenylalanine, tryptophan and histidine) with the non-polar amino acid glycine resulted in a remarkable reduction of the antibody adsorption on the 96 well plate of between 60 to 70 % compared to 35 to 45 % in the presence of arginine alone (4 ng/ml antigen). Processing of the anti-IL6-ELISA with the higher antigen concentration (6 ng/ml) resulted in a stronger reduction of the antibody adsorption between 60 to 80 % compared to 40 to 50 % in the presence of arginine alone (Figure 4). It should be noted, that due to the limited solubility of the aromatic amino acids only the liquid compositions containing 50 mM phenylalanine and 50 mM glycine as well as 50 mM histidine and 50 mM glycine were prepared in equimolar concentrations. In all other liquid compositions, the non-polar osmolytic amino acid glycine was present in a molar excess. Nevertheless, for all liquid compositions the observed effectivity against protein surface adsorption was stronger in comparison to arginine alone and to the amino acid mixtures of arginine in combination with the aromatic amino acids (Example 1.3; Figure 3). Moreover, the positive protein adsorption reducing effectivity observed for glycine in combination with arginine in example 1.2 was confirmed in example 1.4 in combination with aromatic amino acids. Thus, these results clearly evidence that solution comprising two amino acids are able to remarkably reduce the adsorption of the ani-IL6 coating antibody on the well surface better than arginine alone at the same total amino acid concentration in accordance with the claimed invention. Even the strong reduced molar concentrations of the aromatic amino acids due to their limited solubility in the combinations with arginine and with glycine did not negatively influence the effectivity against protein adsorption.

Figure 4: % Reduction of the 450 nm absorption absorption of the anti-IL6 coating antibody onto a polystyrene medium binding surface in the presence of 100 mM Arg*HCl (white bars) as well as 500 mM Arg*HCl (gray bars) and combinations of the aromatic amino acids phenylalanine, tryptophan and histidine with non-polar amino acid glycine at total concentrations of 100 mM (white bars) and 500 mM (gray bars), respectively. The concentration of the coating anti-IL6-antibody was 1.5 µg/ml and the IL6 antigen concentration was 4 ng/ml in (A) and 6 ng/ml in (B). The dashed lines represent the % reduction of the surface adsorption in the presence of Arg*HCl. The positive control for maximal reduction of anti-IL6 coating antibody adsorption (approx. 100 %) is Blocking Buffer (black bars). The quantities for the % reduction of the adsorption of the anti-IL6 coating antibody are depicted on top of the bars in %.

Example 1.5: Reduction of the surface adsorption on polystyrene by a low ionic strength liquid composition comprising combinations of the aromatic amino acids phenylalanine, tryptophan and histidine and the polar amino acid serine.

To investigate the influence of high ionic strength on the protein adsorption induced by the dissolution of the excipients in the high ionic strength buffer PBS, the liquid compositions for this example were prepared by dissolution of the excipients in the low ionic strength buffer 10 mM sodium phosphate pH 7.4. Similar to Example 3 the liquid compositions were prepared in buffer in order to ensure the stability of the coating antibody during the 17 h incubation time at 2-8 °C during the coating step.

Amino acids were provided in equimolar concentration ratios in some samples, e.g. 50 mM: 50 mM in the case of the total amino acid concentration of 100 mM and 250 mM : 250 mM for the total amino acid concentration of 500 mM. Due to the limited solubility of the aromatic amino acids phenylalanine, tryptophan and histidine the mixtures with serine particularly for the high total amino acid concentration of 500 mM were prepared containing non-equimolar concentration ratios. In the case of the low molar concentration of 100 mM only the liquid composition comprised tryptophan and serine in non-equimolar concentrations. Accordingly, the liquid compositions comprised the polar non-charged amino acid serine in a molar excess concentration. The following solutions were prepared:
Arg*HCl (500 mM) without other additives
Arg*HCl (100 mM) without other additives
Phe/Ser (500 mM) = 125 mM Phe + 375 mM Ser
Phe/Ser (100 mM) = 50 mM Phe + 50 mM Ser
Trp/Ser (500 mM) = 35 mM Trp + 465 mM Ser
Trp/Ser (100 mM) = 25 mM Trp + 75 mM Ser
His/Ser (500 mM) = 175 mM His + 325 mM Ser
His/Ser (100 mM) = 50 mM His + 50 mM Ser

As shown in Figure 5, comparable results were observed for combinations of aromatic amino acids (phenylalanine, tryptophan and histidine) with the polar uncharged amino acid serine to the results for the combinations of aromatic amino acids with the non-polar amino acid glycine. These combinations resulted in a % reduction of the antibody adsorption between 50 to 72 % (4 ng/ml antigen) and 60 to 70 % (6 ng/ml) compared to 17 to 35 % and 30 to 45 % in the case of arginine alone (Figure 5).

The combination of aromatic amino acids with the polar amino acid serine seems to have a slightly lower efficacy in the reduction of surface adsorption of the anti-IL6 coating antibody in comparison to the non-polar glycine in combination with the aromatic amino acids (Figure 4 and 5). This effect may be a result of the low ionic strength buffer in the liquid compositions or of the different physico-chemical properties of the two different amino acids glycine and serine. In contrast to the effect of arginine alone which seems to be lower at 100 mM compared to 500 mM in the low ionic strength buffer, the effect of the mixtures of aromatic amino acids and the polar non-charged amino acid serine was relatively independent of the total amino acid concentration.

Example 1.6: Reduction of the surface adsorption on polystyrene in the presence of liquid compositions comprising combinations of the aromatic amino acids phenylalanine, tryptophan and histidine and the negatively charged amino acids glutamic acid and aspartic acid.

As discussed in the previous examples, the addition of the non-polar osmolytic amino acid glycine and the polar non-charged amino acid serine in a molar excess over aromatic amino acids phenylalanine, tryptophan and histidine characterised by a limited water-solubility resulted in a remarkable reduction of protein adsorption on hydrophobic polystyrene medium binding plates.

To test other amino acid groups concerning the physico-chemical properties of the side chains combinations of the aromatic amino acids phenylalanine, tryptophan and histidine with the negatively charged amino acids glutamic acid and aspartic acid were analysed. Due to the limited water-solubility of both kinds of amino acids, the liquid compositions were prepared with remarkable lower total amino acid concentrations. Thus, the molar concentration ratios of the two amino acids were different to the liquid compositions tested in the above experiments and the total amino acid concentrations were significantly smaller compared to the arginine concentrations of 100 mM and 500 mM arginine. In the case of the higher total amino acid concentrations, the concentration of the aromatic amino acids were comparable to the liquid compositions according to the last example. The negatively charged amino acids were added in concentrations according to their maximal water-solubility. All liquid compositions were prepared in the low ionic strength buffer 10 mM sodium phosphate at pH 7.4 as follows:
Trp/Glu (85 mM) = 35 mM Trp + 50 mM Glu
Trp/Glu (42.5 mM) = 17.5 mM Trp + 25 mM Glu
His/Glu (225 mM) = 175 mM His + 50 mM Glu
His/Glu (112.5 mM) = 62.5 mM His + 50 mM Glu
Phe/Glu (175 mM) = 125 mM Phe + 50 mM Glu
Phe/Glu (87.5 mM) = 43.75 mM Phe + 43.75 mM Glu
Trp/Asp (55 mM) = 35 mM Trp + 20 mM Asp
Trp/Asp (27.5 mM) = 13.75 mM Trp + 13.75 mM Asp
His/Asp (195 mM) = 175 mM His + 20 mM Asp
His/Asp (97.5 mM) = 77.5 mM His + 20 mM Asp
Phe/Asp (145 mM) = 125 mM Phe + 20 mM Asp
Phe/Asp (72.5 mM) = 52.5 mM Phe + 20 mM Asp

Surprisingly, the addition of negatively charged amino acids to aromatic amino acids in liquid compositions at a lower total amino acid concentration compared to arginine alone with a molar concentration of 100 mM and 500 mM resulted in a comparable or even higher reduction of protein adsorption on the plate surface.

As shown in Figure 6, the combination of glutamic acid as well as aspartic acid with tryptophan with total amino acid concentrations of 85 mM (Trp/Glu) and 55 mM (Trp/Asp) led to a reduction between 50 % and 60 % compared to 43 % to 51 % compared to arginine alone. At the lower antigen concentration (4 ng/mL) the liquid compositions containing combinations of histidine with glutamic acid (total amino acid concentrations: 225 mM; 112.5 mM) and aspartic acid (total amino acid concentrations: 195 mM; 97.5 mM) revealed a more or less comparable reduction of protein adsorption in comparison to arginine (100 mM; 500 mM; Figure 6). At low antigen concentrations (4 ng/ml), the analysed combinations of phenylalanine with glutamic acid (175 mM) as well as aspartic acid (145 mM; 72.5 mM) exhibited a comparable, or slightly higher reduction of surface adsorption compared to arginine. Thus, the results of this example provide evidence that combinations of aromatic amino acids with negatively charged amino acids combined in remarkably lower total amino acid concentrations can lead to a higher or comparable effectivity against protein surface adsorption in comparison to higher concentrations of arginine alone.

Example 1.7: Reduction of the surface adsorption on polystyrene in the presence of liquid compositions comprising combinations of three amino acids.

In the next step, the effectivity in reducing protein adsorption of combinations of three different amino acids was analyzed by combining the liquid compositions according to the previous examples. Mixtures of aromatic amino acids with the polar non-charged amino acid serine and the negatively charged amino acid glutamic acid were prepared in 10 mM sodium phosphate buffer at pH 7.4. The total amino acid concentrations were adjusted to 100 mM and 500 mM in mainly equimolar concentration ratios with the exception for excipients with low solubility. In the case of high total amino acid concentration of 500 mM, the amino acid mixtures were not equimolar due to the limited solubilities of the aromatic amino acids and glutamic acid. Therefore, the polar amino acid serine was comprised in all liquid compositions with the highest total amino acid concentration as the amino acid with the molar excess. Amino acid mixtures with a total amino acid concentration of 100 mM were all equimolar. The applied liquid compositions contained the three amino acids were prepared with the following molar ratios:
Arg*HCl (500 mM) without other additives
Arg*HCl (100 mM) without other additives
Phe/Ser/Glu (500 mM) = 125 mM Phe + 325 mM Ser + 50 mM Glu
Phe/Ser/Glu (100 mM) = 33.33 mM Phe + 33.33 mM Ser + 33.33 Glu
Trp/Ser/Glu (500 mM) = 35 mM Trp + 415 mM Ser + 50 mM Glu
Trp/Ser/Glu (100 mM) = 33.33 mM Trp + 33.33 mM Ser + 33.33 mM Glu
His/Ser/Glu (500 mM) = 175 mM His + 275 mM Ser + 50 mM Glu
His/Ser/Glu (100 mM) = 33.33 mM His + 33.33 mM Ser + 33.33 mM Glu

As shown in Figure 7, the addition of the negatively charged amino acid Glu slightly reduced the effectivity of the mixture of the aromatic amino acids in combination with the polar uncharged amino acid serine analyzed in Example 1.5 (Figure 5). This effect of the negatively charged amino acid glutamic acid may be a result of the negative charge of this amino acid. Notably, mixtures of three amino acids as analyzed showed a remarkable reduction of protein adsorption compared to the liquid compositions of the negatively charged amino acids in combination with the aromatic amino acids without the polar uncharged amino acid serine (Example 1.6; Figure 6). In summary, the reduction of protein adsorption in the presence of said mixtures comprising three different amino acids was still remarkably higher as compared to arginine alone in both concentrations.

In order to increase the effectivity of the mixtures containing three different amino acids the polar uncharged amino acid serine was substituted by the non-polar small osmolytic amino acid glycine, leading to the combinations of aromatic amino acids with the non-polar osmolytic amino acid glycine and the negatively charged amino acid glutamic acid. The mixtures were prepared in the low ionic strength 10 mM sodium phosphate buffer at pH 7.4 similar to the previous compositions in this example. Total amino acid concentrations of 100 mM and 500 mM were prepared. Due to the limited solubility of the aromatic amino acids liquid compositions with a total amino acid concentration of 500 mM comprised non-equimolar mixtures of the three amino acids. Mixtures with a total amino acid concentration of 100 mM were prepared as equimolar amino acid mixtures. The non-polar hydrophobic amino acid was present in the 500 mM liquid compositions in a molar excess. The concentrations of the single amino acids are comparable to the mixtures of aromatic amino acids/serine/glutamic acid. The prepared liquid compositions comprised the following molar concentration ratios of the amino acids:
Arg*HCl (500 mM) without other additives
Arg*HCl (100 mM) without other additives
Trp/Gly/Glu (500 mM) = 35 mM Trp + 415 mM Gly + 50 mM Glu
Trp/Gly/Glu (100 mM) = 33.33 mM Trp + 33.33 mM + 33.33 mM Glu
His/Gly/Glu (500 mM) = 175 mM His + 275 mM Gly + 50 mM Glu
His/Gly/Glu (100 mM) = 33.33 mM His + 33.33 mM Gly + 33.33 mM Glu
Phe/Gly/Glu (500 mM) = 125 mM Phe + 325 mM Gly + 50 mM Glu
Phe/Gly/Glu (100 mM) = 33.33 mM Phe + 33.33 mM Gly + 33.33 mM Glu

As shown in Figure 8, the substitution of serine by glycine in the liquid compositions of three amino acids further containing aromatic amino acids and the negatively charged amino acid glutamic acid resulted in an increased reduction of the adsorption of the anti-IL6 coating antibody on plate surface. A reduction of protein adsorption between 37 % to 65 % for the total amino acid concentration of 100 mM and between 61 % to 78 % for the total amino acid concentration of 500 mM compared to 18 % - 22 % and 25 % for arginine alone was observed. The addition of the non-polar osmolytic amino acid glycine to the mixtures of aromatic amino acids and the negatively charged amino acid glutamic acid revealed a remarkably increased ability of the liquid compositions to reduce protein surface adsorption (see Example 1.6; Figure 6). The results of Example 1.6 substantiated the claimed invention regarding the effectivity of three different amino acid against protein adsorption stronger than arginine alone and mainly comparable to the liquid compositions containing 2 amino acids with and without arginine.

Example 1.8: Reduction of the surface adsorption on polypropylen in the presence of liquid compositions comprising combinations of aromatic amino acids phenylalanine, tryptophane and histidine with the non-polar hydrophobic, branched amino acid leucine.

As many primary packaging systems for biological pharmaceutics, e.g. syringes, bags etc. are manufactured using polypropylene as material, we analyzed the effectivity of several amino acid mixtures against the adsorption of the anti-IL6 coating antibody on the hydrophobic surface polypropylene in addition to polystyrene plates used in experiments 1.2 to 1.7. In this example, combinations of aromatic amino acids phenylalanine, tryptophan and histidine with the hydrophobic non-polar branched chain amino acid leucine were analyzed.

Due to the limited water-solubility of non-polar hydrophobic and aromatic amino acids, the liquid compositions were prepared with remarkable lower total amino acid concentration. Concentration ratios of the two amino acids were different compared to the previous examples and the total amino acid concentrations were smaller than 100 mM and 500 mM arginine. In the case of the higher molar concentrations, the molar concentration of the containing aromatic amino acids were comparable to the liquid compositions according to the previous example.

The non-polar hydrophobic, branched amino acid was added in molar concentrations according to their maximal water-solubility. All liquid compositions were prepared in the low ionic strength buffer 10 mM sodium phosphate at pH 7.4 as follows:
Trp/Leu (135 mM) = 35 mM Trp + 100 mM Leu
Trp/Leu (67.5 mM) = 33.75 mM Trp + 33.75 mM Leu
His/Leu (275 mM) = 175 mM His + 100 mM Leu
His/Leu (137.5 mM) = 68.75 mM His + 68.75 mM Leu
Phe/Leu (225 mM) = 125 mM Phe + 100 mM Leu
Phe/Leu (112.5 mM) = 56.25 mM Phe + 56.25 mM Leu

As shown in Figure 9, the combinations of aromatic amino acids with the non-polar hydrophobic, branched chain amino acid leucine in remarkably lower total amino acid concentration in comparison the 100 and 500 mM arginine revealed higher or comparable effectivities to reduce protein adsorption on the polypropylene surface in some cases. Particularly, the combination of histidine with leucine resulted in reductions of the adsorption of the anti-IL6 coating antibody around 50 %. Not driven by any theory, these results suggest that the hydrophobic amino acid leucine as well as the hydrophobic aromatic parts of the aromatic amino acids may saturate the hydrophobic surface and so reduce the adsorption of the anti-IL6 coating antibody on the surface. The effectivity of arginine could also be results of interactions between the hydrophobic CH₂ chain in the side chain and the hydrophobic surface. This may explain that low concentrations of arginine are not effective against protein adsorption on this kind of plates.

Example 1.9: Reduction of the surface adsorption on polypropylen in the presence of liquid compositions comprising combinations of aromatic amino acids phenylalanine, tryptophan and histidine with the negatively charged amino acids glutamic acid and aspartic acid.

Amino acid mixtures were prepared analogous to the similar mixtures according to Example 1.6. Due to the limited water-solubility of the amino acids used, the liquid compositions were prepared with remarkable lower total amino acid concentrations. Thus, the molar concentration ratios of the two amino acids were remarkably different to the previous liquid compositions and the total amino acid concentrations were remarkably smaller than the arginine concentrations of 100 mM and 500 mM. For the higher molar concentrations, the molar concentration of the comprised aromatic amino acids were comparable to the liquid compositions according to the previous example. The negatively charged amino acids were added in molar concentrations according to their maximal water-solubility. All liquid compositions were prepared in the low ionic strength buffer 10 mM sodium phosphate at pH 7.4 as follows:
Trp/Glu (85 mM) = 35 mM Trp + 50 mM Glu
Trp/Glu (42.5 mM) = 17.5 mM Trp + 25 mM Glu
His/Glu (225 mM) = 175 mM His + 50 mM Glu
His/Glu (112.5 mM) = 62.5 mM His + 50 mM Glu
Phe/Glu (175 mM) = 125 mM Phe + 50 mM Glu
Phe/Glu (87.5 mM) = 43.75 mM Phe + 43.75 mM Glu
Trp/Asp (55 mM) = 35 mM Trp + 20 mM Asp
Trp/Asp (27.5 mM) = 13.75 mM Trp + 13.75 mM Asp
His/Asp (195 mM) = 175 mM His + 20 mM Asp
His/Asp (97.5 mM) = 77.5 mM His + 20 mM Asp
Phe/Asp (145 mM) = 125 mM Phe + 20 mM Asp
Phe/Asp (72.5 mM) = 52.5 mM Phe + 20 mM Asp

As shown in Figure 10 the presence of the amino acid combinations of aromatic amino acids with negatively charged amino acids glutamic acid and aspartic acid during coating of the anti-IL6-antibody resulted in a comparable or higher reduction of adsorption to the hydrophobic polypropylene surface in comparison to arginine (Figure 10). At 100 mM the effectivity of arginine against protein adsorption to the polypropylene surface is very low, in line with the experiment according to example 1.8 (Figure 9 and 10). The negatively charged amino acid aspartic acid is more effective against protein adsorption onto the polypropylene surface than glutamic acid in combination with the aromatic amino acids (Figure 10).

## Claims

1. A method of reducing or preventing the adsorption of a polypeptide on a surface comprising the steps of:
a) providing a composition comprising the polypeptide and at least two different amino acids in a total amino acid concentration from 10 mM to 550 mM; where the composition comprises at least two different amino acids wherein at least one amino acid is selected from group (d) of amino acids with negatively charged R groups; and further comprises at least one amino acid selected is from group (e) of amino acids with aromatic R groups; and
b) contacting the composition with the surface,
wherein the surface comprises or consists of a metal, a glass or a polymer material, preferably selected from polyethylene (PE), for example selected from polyethylene terephthalate (PET), high density polyethylene (HDPE), low-density polyethylene (LDPE), linear low density polyethylene (LLDPE), polypropylene (PP), polystyrene (PS), polyvinylchloride (PVC), polyvinylidene chloride (PVDC), polytetrafluorethylene (PTFE), polyethersulphone (PES), polymethylmethacrylate, polycarbonate (PC, bisphenol A), nylon, polyether urethane, polysiloxane (silicone), polychlorotrifluoroethylene (PCTFE)/PVC laminates, polyamide (PA), e.g. orientated polyamide (OPA), cyclic olefin copolymer, or mixtures and copolymers thereof.

2. The method of claim 1, wherein the composition comprises at least five amino acids from five different groups.

3. The method of any of the preceding claims, wherein the composition is an aqueous solution.

4. The method of any of the preceding claims, wherein the composition is free or substantially free of at least one stabilising protein and/or wherein the composition is free or substantially free of at least one surfactant.

5. The method of any of the preceding claims, wherein the polypeptide concentration in the composition is at least 100 mg/ml, preferably at least 150 mg/ml.

6. The method of any of claims 1 to 5, wherein the polypeptide concentration in the composition is not higher than 20 mg/ml, preferably not higher than 5 mg/ml.

7. The method of any of the preceding claims, wherein the polypeptide is comprised or attached to the capsid or envelope of a virus or a viral vector.

8. The method of any of the preceding claims, wherein the surface is a hydrophobic surface.

9. The method of any of the preceding claims, wherein the surface is part of a medical device.

10. The method according to any one of claims 1 to 9, wherein the composition comprises at least three different amino acids wherein the at least three amino acids are at least one amino acid selected from group (b) of amino acids a polar uncharged R, or at least one amino acid from group (a) of amino acids with an unpolar aliphatic R group, and further at least one amino acid is selected from group (d) of amino acids with negatively charged R groups; and at least one amino acid is selected from group (e) of amino acids with aromatic R groups.

11. The method according to any one of claims 1 to 9, wherein the composition comprises at least two different amino acids wherein at least one amino acid is selected from any of the groups of amino acids with (a) of amino acids with an unpolar aliphatic R group (b) of amino acids with a polar uncharged R group;(c) of amino acids with positively charged R groups; and/or (d) of amino acids with negatively charged R groups; and further comprises at least one amino acid selected is from group (e) of amino acids with aromatic R groups.

## Patentansprüche

1. Verfahren zur Verringerung oder Vermeidung der Adsorption eines Polypeptids an eine Oberfläche, umfassend die folgenden Schritte:
(a) Bereitstellen einer Zusammensetzung, umfassend das Polypeptid und mindestens zwei unterschiedliche Aminosäuren in einer Gesamtaminosäurekonzentration von 10 mM bis 550 mM; wobei die Zusammensetzung mindestens zwei verschiedene Aminosäuren umfasst, wobei mindestens eine Aminosäure aus der Gruppe (d) von Aminosäuren mit negativ geladenen R-Gruppen ausgewählt ist; und weiterhin mindestens eine Aminosäure umfasst, die aus der Gruppe (e) der Aminosäuren mit aromatischen R-Gruppen ausgewählt ist; und
(b) Inkontaktbringen der Zusammensetzung mit der Oberfläche,
wobei die Oberfläche ein Metall, ein Glas oder ein Polymermaterial umfasst oder daraus besteht, vorzugsweise ausgewählt aus Polyethylen (PE), beispielsweise ausgewählt aus Polyethylenterephthalat (PET), Polyethylen hoher Dichte (HDPE), Polyethylen niedriger Dichte (LDPE), linearem Polyethylen niedriger Dichte (LLDPE), Polypropylen (PP), Polystyrol (PS), Polyvinylchlorid (PVC), Polyvinylidenchlorid (PVDC), Polytetrafluorethylen (PTFE), Polyethersulfon (PES), Polymethylmethacrylat, Polycarbonat (PC, Bisphenol A), Nylon, Polyetherurethan, Polysiloxan (Silikon), Polychlortrifluorethylen (PCTFE)/PVC-Laminate, Polyamid (PA), z.B. orientiertes Polyamid (OPA), cyclisches Olefincopolymer oder Gemische und Copolymere davon.

2. Verfahren nach Anspruch 1, wobei die Zusammensetzung mindestens fünf Aminosäuren aus fünf verschiedenen Gruppen umfasst.

3. Verfahren nach einem der vorherigen Ansprüche, wobei die Zusammensetzung eine wässrige Lösung ist.

4. Verfahren nach einem der vorherigen Ansprüche, wobei die Zusammensetzung frei oder im Wesentlichen frei von mindestens einem stabilisierenden Protein ist und/oder wobei die Zusammensetzung frei oder im Wesentlichen frei von mindestens einer oberflächenaktiven Substanz ist.

5. Verfahren nach einem der vorherigen Ansprüche, wobei die Polypeptidkonzentration in der Zusammensetzung mindestens 100 mg/mL ist, vorzugsweise mindestens 150 mg/mL.

6. Verfahren nach einem der Ansprüche 1 bis 5, wobei die Polypeptidkonzentration in der Zusammensetzung nicht höher als 20 mg/mL ist, vorzugsweise nicht höher als 5 mg/mL.

7. Verfahren nach einem der vorherigen Ansprüche, wobei das Polypeptid in dem Kapsid oder der Hülle eines Virus oder eines viralen Vektors enthalten ist oder daran gebunden ist.

8. Verfahren nach einem der vorherigen Ansprüche, wobei die Oberfläche eine hydrophobe Oberfläche ist.

9. Verfahren nach einem der vorherigen Ansprüche, wobei die Oberfläche Teil einer medizinischen Vorrichtung ist.

10. Verfahren nach einem der Ansprüche 1 bis 9, wobei die Zusammensetzung mindestens drei verschiedene Aminosäuren umfasst, wobei die mindestens drei verschiedenen Aminosäuren mindestens eine Aminosäure aus Gruppe (b) der Aminosäuren mit einer polaren ungeladenen R-Gruppe oder mindestens eine Aminosäure aus Gruppe (a) der Aminosäuren mit einer unpolaren aliphatischen R-Gruppe, und weiterhin mindestens eine Aminosäure aus Gruppe (d) der Aminosäuren mit negativ geladenen R Gruppen ausgewählt sind; und mindestens eine Aminosäure aus Gruppe (e) der Aminosäuren mit aromatischen R-Gruppen ausgewählt ist.

11. Verfahren nach einem der Ansprüche 1 bis 9, wobei die Zusammensetzung mindestens zwei unterschiedliche Aminosäuren umfasst, wobei mindestens eine Aminosäure aus einer beliebigen der Gruppen der Aminosäuren mit (a) den Aminosäuren mit einer unpolaren aliphatischen R-Gruppe; (b) den Aminosäuren mit einer polar ungeladenen R-Gruppe; (c) den Aminosäuren mit positiv geladenen R-Gruppen; und/oder (d) den Aminosäuren mit negativ geladenen R-Gruppen ausgewählt ist; und weiterhin mindestens eine Aminosäure aus Gruppe (e) der Aminosäuren mit aromatischen R-Gruppen umfasst.

## Revendications

1. Méthode de réduction ou de prévention de l'adsorption d'un polypeptide sur une surface, comprenant les étapes de :
a) obtention d'une composition comprenant le polypeptide et au moins deux acides aminés différents à une concentration totale des acides aminés de 10 mM à 550 mM ; où la composition comprend au moins deux acides aminés différents, dans laquelle au moins un acide aminé est choisi dans le groupe (d) des acides aminés ayant des groupes R chargés négativement ; et comprend en outre au moins un acide aminé choisi dans le groupe (e) des acides aminés ayant des groupes R aromatiques ; et
b) mise en contact de la composition avec la surface,
dans laquelle la surface comprend ou consiste en un métal, un verre ou un matériau polymère, de préférence choisi parmi un polyéthylène (PE), par exemple choisi parmi un poly(téréphtalate d'éthylène) (PET), un polyéthylène haute densité (HDPE), un polyéthylène basse densité (LDPE), un polyéthylène basse densité linéaire (LLDPE), un polypropylène (PP), un polystyrène (PS), un poly(chlorure de vinyle) (PVC), un poly(chlorure de vinylidène) (PVDC), un polytétrafluoroéthylène (PTFE), une poly(éther-sulfone) (PES), un poly(méthacrylate de méthyle), un polycarbonate (PC, bisphénol A), un nylon, un poly(éther-uréthane), un polysiloxane (silicone), les stratifiés de polychlorotrifluoroéthylène (PCTFE)/PVC, un polyamide (PA), par exemple un polyamide orienté (OPA), un copolymère d'oléfine cyclique, ou leurs mélanges et copolymères.

2. Méthode selon la revendication 1, dans laquelle la composition comprend au moins cinq acides aminés provenant de cinq groupes différents.

3. Méthode selon l'une quelconque des revendications précédentes, dans laquelle la composition est une solution aqueuse.

4. Méthode selon l'une quelconque des revendications précédentes, dans laquelle la composition est exempte ou pratiquement exempte d'au moins une protéine stabilisatrice et/ou dans laquelle la composition est exempte ou pratiquement exempte d'au moins un tensioactif.

5. Méthode selon l'une quelconque des revendications précédentes, dans laquelle la concentration de polypeptide dans la composition est d'au moins 100 mg/ml, de préférence d'au moins 150 mg/ml.

6. Méthode selon l'une quelconque des revendications 1 à 5, dans laquelle la concentration de polypeptide dans la composition n'est pas supérieure à 20 mg/ml, de préférence pas supérieure à 5 mg/ml.

7. Méthode selon l'une quelconque des revendications précédentes, dans laquelle le polypeptide est compris dans ou attaché à la capside ou à l'enveloppe d'un virus ou d'un vecteur viral.

8. Méthode selon l'une quelconque des revendications précédentes, dans laquelle la surface est une surface hydrophobe.

9. Méthode selon l'une quelconque des revendications précédentes, dans laquelle la surface constitue une partie d'un dispositif médical.

10. Méthode selon l'une quelconque des revendications 1 à 9, dans laquelle la composition comprend au moins trois acides aminés différents, dans laquelle les au moins trois acides aminés sont au moins un acide aminé choisi dans le groupe (b) des acides aminés ayant un groupe R polaire non chargé, ou au moins un acide aminé choisi dans le groupe (a) des acides aminés ayant un groupe R aliphatique non polaire, et en outre au moins un acide aminé choisi dans le groupe (d) des acides aminés ayant des groupes R chargés négativement ; et au moins un acide aminé choisi dans le groupe (e) des acides aminés ayant des groupes R aromatiques.

11. Méthode selon l'une quelconque des revendications 1 à 9, dans laquelle la composition comprend au moins deux acides aminés différents, dans laquelle au moins un acide aminé est choisi dans l'un quelconque des groupes d'acides aminés parmi (a) des acides aminés ayant un groupe R aliphatique non polaire, (b) des acides aminés ayant un groupe R polaire non chargé ; (c) des acides aminés ayant des groupes R chargés positivement ; et/ou (d) des acides aminés ayant des groupes R chargés négativement ; et comprend en outre au moins un acide aminé choisi dans le groupe (e) des acides aminés ayant des groupes R aromatiques.
